# EUROPEAN PATENT SPECIFICATION

(11) **EP 3 231 868 B1**
(45) Date of publication and mention of the grant of the patent: **27.05.2020**
(21) Application number: 15867235.2
(22) Date of filing: 07.09.2015
(51) Int. Cl.: C12N 15/11, C12N 15/63, A61K 48/00, A61P 35/00

(54) **O-ANTIGEN CARBOHYDRATE CHAIN EXTENDED SALMONELLA PARATYPHI A AND USE THEREOF**
O-ANTIGEN-KOHLENHYDRATKETTENVERLÄNGERTES SALMONELLA PARATYPHI A UND VERWENDUNG DAVON
SALMONELLA PARATYPHI A À CHAÎNE HYDROCARBONÉE D'ANTIGÈNE O ALLONGÉE ET UTILISATION ASSOCIÉE

(30) Priority: 08.12.2014 CN 201410743690
(43) Date of publication of application: 18.10.2017
(73) Proprietor: Institute of Biotechnology, Academy of Military Medical Sciences, China, Beijing 100071 (CN)
(72) Inventor: WU, Jun, Beijing (CN); SUN, Peng, Beijing (CN); LIU, Bo, Beijing (CN); CHANG, Shaohong, Beijing (CN); GONG, Xin, Beijing (CN); WANG, Hengliang, Beijing (CN); ZHU, Li, Beijing (CN); PAN, Chao, Beijing (CN); FENG, Erling, Beijing (CN)
(74) Representative: Weickmann & Weickmann PartmbB
(86) International application number: PCT/CN2015/089045
(87) International publication number: WO 2016/090980

(56) References cited:
- EP-A1- 3 225 690
- CN-A- 1 448 504
- CN-A- 102 140 430
- F. MICOLI ET AL: "A scalable method for O-antigen purification applied to various Salmonella serovars", ANALYTICAL BIOCHEMISTRY, vol. 434, no. 1, 1 March 2013 (2013-03-01) , pages 136-145, XP055466360, AMSTERDAM, NL ISSN: 0003-2697, DOI: 10.1016/j.ab.2012.10.038
- Julian Ihssen ET AL: "Production of glycoprotein vaccines in Escherichia coli", , 1 January 2010 (2010-01-01), XP055158778, DOI: 10.1186/1475-2859-9-61 Retrieved from the Internet: URL:http://www.microbialcellfactories.com/ content/pdf/1475-2859-9-61.pdf [retrieved on 2014-12-15]
- FARIDMOAYER A ET AL: "Extreme substrate promiscuity of the Neisseria oligosaccharyl transferase involved in protein O-glycosylation", JOURNAL OF BIOLOGICAL CHEMISTRY, AMERICAN SOCIETY FOR BIOCHEMISTRY AND MOLECULAR BIOLOGY, US, vol. 283, no. 50, 12 December 2008 (2008-12-12), pages 34596-34604, XP002616754, ISSN: 0021-9258, DOI: 10.1074/JBC.M807113200
- G. L. MURRAY ET AL: "Altering the Length of the Lipopolysaccharide O Antigen Has an Impact on the Interaction of Salmonella enterica Serovar Typhimurium with Macrophages and Complement", JOURNAL OF BACTERIOLOGY, vol. 188, no. 7, 1 April 2006 (2006-04-01) , pages 2735-2739, XP55466150, US ISSN: 0021-9193, DOI: 10.1128/JB.188.7.2735-2739.2006
- DATSENKO KIRILL A ET AL: "One-step inactivation of chromosomal genes in Escherichia coli K-12 using PCR products", PROCEEDINGS NATIONAL ACADEMY OF SCIENCES PNAS, NATIONAL ACADEMY OF SCIENCES, US, vol. 97, no. 12, 6 June 2000 (2000-06-06), pages 6640-6645, XP002210218, ISSN: 0027-8424, DOI: 10.1073/PNAS.120163297
- CHAVEROCHE M K ET AL: "A rapid method for efficient gene replacement in the filamentous fungus Aspergillus nidulans", NUCLEIC ACIDS RESEARCH, INFORMATION RETRIEVAL LTD, vol. 28, no. 22 , - 2000, page E97, XP002371804, ISSN: 0305-1048, DOI: 10.1093/NAR/28.22.E97
- CHAO PAN ET AL: "ABSTRACT", MBIO, vol. 7, no. 2, 26 April 2016 (2016-04-26), pages e00443-16, XP055465797, DOI: 10.1128/mBio.00443-16
- PENG SUN ET AL: "Design and production of conjugate vaccines against S. Paratyphi A using an O-linked glycosylation system in vivo", NPJ VACCINES, vol. 3, no. 1, 1 January 2018 (2018-01-01) , XP055466286, DOI: 10.1038/s41541-017-0037-1
- DATABASE UniProt [Online] 27 May 2015 (2015-05-27), "SubName: Full=Chain length determinant protein {ECO:0000313|EMBL:PHK79504.1}; SubName: Full=Chain length determinant protein WzzB {ECO:0000313|EMBL:KTZ12448.1};", XP002779976, retrieved from EBI accession no. UNIPROT:A0A0D6FGW8 Database accession no. A0A0D6FGW8
- COHEN, J.O. ET AL.: 'Identification of Salmonella O antigens by coagglutination' PROGRESS IN MICROBIOLOGY AND IMMUNOLOGY vol. 19, no. 5, 01 May 1984, pages 576 - 578, XP055454097
- SUN, HUALU: 'ISOLATION AND IDENTIFICATION OF SALMONELLA CONSTRUCTION AND B iological Characteristics of Deletion Mutants of Genes for Biofilm Formation from Salmonella Pullorum' AGRICULTURE, CHINA MASTER'S THESES FULL-TEXT DATABASE no. 7, 15 July 2013, pages D050 - D191, XP009503811

## Description

### Technical Field

The present invention relates to the field of biotechnology, and in particular, relates to a *Salmonella Paratyphi A* with an O-Antigen having an extended carbohydrate chain and use thereof.

### Background Art

*Salmonella spp.* is a highly contagious gram-negative intestinal pathogen with strong endotoxin and invasiveness, belongs to intracellular bacteria, and can attach small intestinal mucosal to cause diseases such as enteric fever, gastroenteritis and septicemia, even severe intestinal bleeding or perforation. For most serotypes of *Salmonella,* their median infective doses are between 10⁵ and 10⁸, but in epidemic outbreaks, the infective doses are generally less than 10³ bacteria, sometimes even less than 100 bacteria. In Asian countries, especially in China, the proportion of intestinal diseases caused by *Salmonella paratyphi A* is increasing, and some studies find that there are 150 cases of *Salmonella paratyphi A* infection per 100 000 people per year.

At present, the main way to treat typhoid and paratyphoid is antibiotics, but with the emergence of drug-resistance, especially the emergence of multiple drug-resistant strains, conventional antibiotic treatment encounters a huge challenge, and immunization of relevant vaccines is an effective means of prevention. At present, the progresses of research and development for oral attenuated live vaccine against *Salmonella typhimurium,* Vi capsular polysaccharide vaccine and Vi polysaccharide-protein conjugate vaccine are rapid, and there are a variety of products listed, but these vaccines are not able to generate cross immunoprotection against *Salmonella paratyphi A.* Currently, there is not a vaccine against *Salmonella paratyphi A* that has been approved for listing.

### Contents of the Invention

One object of the present invention is to provide a recombinant strain. The solution to this and further objects of the invention are set forth in the patent claims.

The recombinant strain as provided by the present invention is obtained by introducing *cld*_{LT2} gene encoding an enzyme controlling chain length of O-antigen of *Salmonella typhimurium* into *Salmonella paratyphi A* deficient in *cld* gene encoding an enzyme controlling chain length of O-antigen.

In the above recombinant strain, the enzyme (also named as Cld_{LT2}) controlling chain length of O-antigen of *Salmonella typhimurium* has an amino acid sequence as shown in SEQ ID NO: 2;
and preferably the enzyme Cld_{LT2} controlling chain length of O-antigen of *Salmonella typhimurium* has a coding sequence as shown in SEQ ID NO: 1.

In the above recombinant strain, *Salmonella paratyphi A* deficient in *cld* gene encoding an enzyme controlling chain length of O-antigen is obtained by knocking out *cld* gene via Red recombination technology, or by knocking out *cld* gene via homologous recombination, or by inserting an inactivated *cld* gene, or the *cld deficient* strain can be obtained by induced mutation; preferably, is obtained by knocking out *cld* gene via Red recombination technology.

In the above recombinant strain, *Salmonella paratyphi A* deficient in *cld* gene encoding an enzyme controlling chain length of O-antigen is obtained by a method comprising the following steps:
(1) preparing a linear targeting DNA fragment 1, which has a nucleotide sequence as shown in SEQ ID NO: 11, which contains a cat gene;
(2) transforming pKD46 plasmid into *Salmonella*. *paratyphi*, to obtain a recombinant strain designated as *S. paratyphi*/pKD46;
(3) inducing expression of Red recombination system in the *S. paratyphi*/pKD46 strain, and transforming the linear targeting DNA fragment 1 into the *S. paratyphi*/pKD46 strain, so that the linear targeting DNA fragment 1 replaces the *cld* gene in the *S. paratyphi*/pKD46 strain to obtain a recombinant strain designated as *S. paratyphi cld::cat*/pKD46;
(4) deleting the pKD46 plasmid from the *S. paratyphi cld::cat*/pKD46 strain, to obtain a recombinant strain designated as *S. paratyphi cld::cat*;
   the *S. paratyphi cld::cat* is a *S. paratyphi* in which *cld* gene sequence is substituted with *cat* gene sequence;
(5) transforming plasmid pCP20 into the *S. paratyphi cld::cat* and deleting *cat* gene, to obtain a recombinant strain designated as *S. paratyphi Δcld*;
   the *S. paratyphi Δcld* is a *cld* gene-deleted *S. paratyphi.*

Another object of the present invention is to provide a method for extending carbohydrate chain length of O-antigen of *Salmonella paratyphi A.*

The method for extending carbohydrate chain length of O-antigen of *Salmonella paratyphi A* as provided in the present invention comprises the following steps: culturing the above recombinant strain to express the *cld*_{LT2} gene, so that the recombinant strain synthesizes an O-antigen of which carbohydrate chain length is extended.

It is also an object of the present invention to provide an O-antigen.

The O-antigen is prepared according to the method described above.

The use of the above-described O-antigen in the manufacture of a product for the prevention or prophylaxis of diseases caused by *Salmonella paratyphi A* is also within the scope of the present invention.

It is a further object of the present invention to provide an method for preparing a vaccine for the prevention and/or treatment of diseases caused by *Salmonella paratyphi A* via one-step bio-crosslinking.

The method for preparing a vaccine for the prevention and/or treatment of diseases caused by *Salmonella paratyphi A* as provided in the present invention via one-step bio-crosslinking method comprises the steps of:
(1) inactivating *cld* gene encoding an enzyme controlling chain length of O-antigen of *Salmonella paratyphi A* and *waaL* gene encoding O-antigen ligase of *Salmonella paratyphi A,* to obtain a *Salmonella paratyphi A* with double deletion of *cld* gene and *waaL* gene, wherein said *cld* gene has a sequence as shown by sites from 887812 to 888789 of the sequence with accession number CP000026 in GenBank;
(2) introducing a *cld*_{LT2} gene encoding an enzyme controlling chain length of O-antigen of *Salmonella typhimurium,* a pglL gene encoding O-oligosaccharyltransferase of *Neisseria meningitidis* and a gene encoding recombinant fusion protein into the *Salmonella paratyphi A* with double deletion of *cld* gene and *waaL* gene, to obtain a recombinant strain, wherein said *cld*_{LT2} gene encodes the enzyme controlling chain length of O-antigen of *Salmonella typhimurium* which has an amino acid sequence as shown in SEQ ID NO: 2;
(3) culturing the recombinant strain to obtain an O-antigen-modified recombinant fusion protein and processing the O-antigen-modified recombinant fusion protein to obtain the target vaccine;
   wherein the recombinant fusion protein comprises a N-terminal signal peptide, a carrier protein, and a peptide fragment comprising a serine as an O-glycosylation site at position 63 of *Neisseria meningitidis* pilin PilE;
   the N-terminal signal peptide is PelB signal peptide, DsbA signal peptide, STII signal peptide, OmpA signal peptide, PhoA signal peptide, LamB signal peptide, SpA signal peptide or Enx signal peptide, preferably a DsbA signal peptide;
   the carrier protein is a non-toxic mutant of a bacterial toxin protein or a fragment of a bacterial toxin protein;
   the peptide fragment comprising a serine as an O-glycosylation site at position 63 of *Neisseria meningitidis* pilin PilE is a peptide fragment with an amino acids as set forth in positions 45-73 of *Neisseria meningitidis* pilin PilE.In the above method, the *Salmonella paratyphi A* with double deletion of *cld* gene encoding an enzyme controlling chain length of O-antigen and *waaL* gene encoding O-antigen ligase can be obtain by knocking out *cld* gene and *waaL* gene via Red recombination technology, or by knocking out *cld* gene and *waaL* gene via homologous recombination, or by inserting an inactivated *cld* gene and *waaL* gene, or by obtaining a strain with deletion of *cld* and *waaL* genes by induced mutation; preferably, is obtained via Red recombination technology.

In the above method, the *Salmonella paratyphi A* with double deletion of *cld* gene encoding an enzyme controlling chain length of O-antigen and *waaL* gene encoding O-antigen ligase is constructed by a method comprising the following steps:
(1) preparing a linear targeting DNA fragment 2, which has a nucleotide sequence as shown in SEQ ID NO: 12, which contains a *kan* gene;
(2) transforming pKOBEG plasmid into the *S. Paratyphi*Δ*cld* strain, to obtain a recombinant strain designated as *S. paratyphi*Δ*cld*/pKOBEG;
(3) inducing expression of Red recombination system in the *S. paratyphi*Δ*cld*/pKOBEG strain, and transforming the linear targeting DNA fragment 2 into the *S. paratyphi*Δ*cld*/pKOBEG strain, so that the linear targeting DNA fragment 2 replaces the *waaL* gene in the *S. paratyphi*Δ*cld*/pKOBEG strain to obtain a recombinant strain designated as *S. Paratyphi*Δ*cld waal::kan*/pKOBEG;
(4) deleting the pKOBEG plasmid from the *S. paratyphi*Δ*cld waal::kan*/pKOBEG strain, to obtain a recombinant strain designated as *S. paratyphi*Δ*cldwaaL::kan*;
   the *S. paratyphi*Δ*cldwaaL::kan* is a *S. paratyphi*Δ*cld* in which *waaL* gene sequence is substituted with *kan* gene sequence;
(5) transforming plasmid pCP20 into the *S. paratyphi*Δ*cldwaaL::kan* and deleting *kan* gene, to obtain a recombinant strain designated as *S. paratyphi*Δ*cld*Δ*waaL*;
   the *S. paratyphi*Δ*cld*Δ*waaL* is a *S. paratyphi* with deletion of *cld* gene and *waaL* gene.

In the above method, the bacterial toxin protein is *Pseudomonas aeruginosa* exotoxin A, cholera toxin, diphtheria toxin or tetanus toxin.

In the above method, the non-toxic mutant of the bacterial toxin protein is a non-toxic mutant of *Pseudomonas aeruginosa* exotoxin A or a non-toxic mutant of diphtheria toxin; the fragment of the bacterial toxin protein is a B subunit of cholera toxin or a C protein of tetanus toxin.

In the above method, the carrier protein is specifically a non-toxic mutant of *Pseudomonas aeruginosa* exotoxin A or a B subunit of cholera toxin.

In the above method, the *cld*_{LT2} gene encoding an enzyme controlling chain length of O-antigen of *Salmonella typhimurium,* the *pglL* gene encoding O-oligosaccharide transferase of *Neisseria meningitidis* and a gene encoding the recombinant fusion protein can be separately constructed into different recombinant expression vectors and introduced into the *Salmonella paratyphi A* with double deletion of *cld* and *waaL,* or can be constructed into the same recombinant expression vector and introduced into the *Salmonella paratyphi A* with double deletion of *cld* and *waaL,* or can also be introduced into the *Salmonella paratyphi A* with double deletion of *cld* and *waaL* via separately incorporating them into a host genome; the cld_{LT2} gene encoding an enzyme controlling chain length of O-antigen of *Salmonella typhimurium,* the *pglL* gene encoding O-oligosaccharide transferase of *Neisseria meningitidis* and the gene encoding the recombinant fusion protein can be controlled by an inducible promoter, or controlled by a constitutive promoter.

In the above method, the *cld*_{LT2} gene encoding an enzyme controlling chain length of O-antigen of *Salmonella typhimurium,* the *pglL* gene encoding O-oligosaccharide transferase of *Neisseria meningitidis* are specifically introduced into the *Salmonella paratyphi A* with double deletion of *cld* gene encoding an enzyme controlling chain length of O-antigen and *waaL* gene encoding O-antigen ligase via pETtac28-*pglL*-*cld*_{LT2} recombinant expression vector.

In the above method, the gene encoding the recombinant fusion protein is specifically introduced into the *Salmonella paratyphi A* with double deletion of *cld* gene encoding an enzyme controlling chain length of O-antigen and *waaL* gene encoding O-antigen ligase via pMMB66EH-rCTB4573 recombinant expression vector or pMMB66EH-rEPA4573 recombinant expression vector or pMMB66EH-rCTB4573₃ recombinant expression vector.

In the above method, the pETtac28-*pglL*-*cld*_{LT2} recombinant expression vector is constructed by a method comprising: using restriction endonucleases *Eco*R I and *Hind* III to perform double digestion of *cld*_{LT2} gene encoding an enzyme controlling chain length of O-antigen of *Salmonella typhimurium* and the pMMB66EH vector, ligating to obtain a transition vector pMMB66EH-*cld*_{LT2}; using the transition vector pMMB66EH-*cld*_{LT2} as template, amplifying to obtain an expression cassette of *cld*_{LT2}, using restriction endonuclease *Xba* I and *Xho* I to perform double digestion of the expression cassette of *cld*_{LT2} and pET28a vector, ligating to obtain pETtac28-*cld*_{LT2} vector; using restriction endonucleases *EcoR* I and *Hind* III to perform double digestion of O-oligosaccharide transferase gene *pglL* of *Neisseria meningitidis* and pKK223-3 vector, ligating to obtain pKK223-3-*pglL* vector; using pKK223-3-*pglL* vector as template, amplifying to obtain an expression cassette of PglL; using restriction endonuclease *Bgl* II to perform double digestion of the expression cassette of PglL and the pETtac28-*cld*_{LT2} vector, ligating to obtain the pETtac28-*pglL*-*cld*_{LT2} recombinant expression vector.

In the above method, the pMMB66EH-rEPA4573 recombinant expression vector is constructed by a method comprising: ligating the gene encoding fusion protein rEPA4573 of recombinant *Pseudomonas aeruginosa* exotoxin A into a multiple cloning site of pMMB66EH vector to obtain the pMMB66EH-rEPA4573 recombinant expression vector.

In the above method, the pMMB66EH-rCTB4573 recombinant expression vector is constructed by a method comprising: ligating the gene encoding fusion protein rCTB4573 of recombinant cholera toxin B subunit into a multiple cloning site of pMMB66EH vector to obtain the pMMB66EH-rCTB4573 recombinant expression vector.

In the above method, the pMMB66EH-rCTB4573₃ recombinant expression vector is constructed by a method comprising: ligating the gene encoding fusion protein rCTB4573₃ of recombinant cholera toxin B subunit into a multiple cloning site of pMMB66EH vector to obtain the pMMB66EH-rCTB4573₃ recombinant expression vector.

In the above method, the enzyme Cld_{LT2} controlling chain length of O-antigen of *Salmonella typhimurium* has an amino acid sequence as shown in SEQ ID NO: 2; and gene encoding the enzyme controlling chain length of O-antigen of *Salmonella typhimurium* has a sequence as shown in SEQ ID NO: 1;
the *Neisseria meningitides* oligosaccharyltransferase pglL has an amino acid sequence as shown in SEQ ID NO: 8; the gene encoding *Neisseria meningitides* oligosaccharyltransferase pglL has a sequence as shown in SEQ ID NO: 7;
the fusion protein rEPA4573 of recombinant *Pseudomonas aeruginosa* exotoxin A has an amino acid sequence as shown in SEQ ID NO: 4; the Nos 1-19 from the N-terminal of the sequence shown in SEQ ID NO: 4 are of an amino acid sequence of the DsbA signal peptide; the amino acids at Nos 20-631 are of an amino acid sequence of the non-toxic mutant of *Pseudomonas aeruginosa* exotoxin A; the Nos 632-636 are of a flexible linker sequence; the amino acids at Nos 637-665 are of an amino acid sequence of a peptide at sites 45-73 of *Neisseria meningitidi* spilin PilE (NC_003112.2); the Nos 666-674 are of a flexible linker sequence and a 6× His tag sequence; the fusion protein rEPA4573 of recombinant *Pseudomonas aeruginosa* exotoxin A has a coding sequence as shown in SEQ ID NO: 3;
the fusion protein rCTB4573 of recombinant cholera toxin B subunit has an amino acid sequence as shown in SEQ ID NO: 6; the Nos 1-19 from the N-terminal of the sequence shown in SEQ ID NO: 6 are of an amino acid sequence of the DsbA signal peptide; the Nos 20-122 are of amino acid sequence of cholera toxin B subunit; the Nos 123-127 are of a flexible linker sequence; the Nos 128-156 are of an amino acid sequence of a peptide at sites 45-73 of *Neisseria meningitidis* pilin PilE (NC_003112.2); the Nos 157-166 are of a flexible linker and a 6× His tag sequence; the fusion protein rCTB4573 of recombinant cholera toxin B subunit has a coding sequence as shown in SEQ ID NO: 5;
the fusion protein rCTB4573₃ of recombinant cholera toxin B subunit has an amino acid sequence as shown in SEQ ID NO: 10; the Nos 1-19 from the N-terminal of the sequence shown in SEQ ID NO: 10 are of an amino acid sequence of the DsbA signal peptide; the Nos 20-122 are of amino acid sequence of cholera toxin B subunit; the Nos 123-127 are of a flexible linker sequence; the Nos 128-222 are of an amino acid sequence of 3 repetitive peptides of that at sites 45-73 of *Neisseria meningitidis* pilin PilE (NC_003112.2); the Nos 223-232 are of a flexible linker and a 6× His tag sequence; the fusion protein rCTB4573₃ of recombinant cholera toxin B subunit has a coding sequence as shown in SEQ ID NO: 9;
the peptide as set forth in amino acid positon 45-73 of *Neisseria meningitidis* pilin PilE has an amino acid sequence as shown in SEQ ID NO: 14; and gene encoding the peptide has a sequence as shown in SEQ ID NO: 13.

Conjugate vaccines can be prepared according to the above methods, such conjugate vaccines can be used in prophylaxis and/or treatment of a disease caused by *Salmonella paratyphi A.*

### Brief Description of the Drawings

Fig.1 shows a PCR identification diagram of *S. paratyphi* CMCC50973 with knockout of *cld* gene.
Fig.2 shows a silver staining diagram of LPS before and after *S. paratyphi* CMCC50973 *cld* gene is replaced.
Fig.3 shows a detection diagram of glycosylation modification of the recombinant fusion protein after the substitution of *cld* gene.
Fig.4 shows a detection diagram of multi-site glycosylation modification of the recombinant CTB fusion proteins.
Fig.5 shows a detection diagram of rEPA4573-OPS_{*Spty*50973} purification effect.
Fig.6 shows a detection diagram of rCTB4573₃-OPS_{*Sp*50973} purification effect.
Fig.7 shows an immunogenicity evaluation of glycoprotein conjugates.
Fig.8 shows a molecular identification of *S. paratyphi* CMCC50973Δ*cld*Δ*waaL.*

### Best Models for Carrying Out the Invention

The experimental methods used in the following examples are conventional methods unless otherwise specified.

The materials, reagents and the like used in the following examples are commercially available, unless otherwise specified.

Experimental materials: *Salmonella paratyphi A* CMCC50973 strain (*Salmonella.paratyphi* CMCC50973), purchased from the China Medical Bacteria Deposit Management Center.

pET-22b plasmid, purchased from Novagen Corporation under catalog number 69744.

pKOBEG plasmid, disclosed in the literature "A rapid method for efficient gene replacement in the filamentous fungus Aspergillus nidulans [J]. Nucleic Acids Res. 2000 Nov 15; 28 (22): E97", available for the public in the Institute of Bioengineering of the Academy of Military Medical Sciences of the Chinese People's Liberation Army; the plasmid is a temperature-sensitive plasmid with chloramphenicol resistance.

pKD3 plasmid, disclosed in the literature "Datsenko, K.A. and B.L. Wanner, One-step inactivation of chromosomal genes in Escherichia coli K-12 using PCR products. Proc Natl Acad Sci USA, 2000,97(12): p. 6640-5."; available for the public in the Institute of Bioengineering of the Academy of Military Medical Sciences of the Chinese People's Liberation Army.

pKD46 plasmid, discloses in the literature "Datsenko, K.A. and B.L. Wanner, One-step inactivation of chromosomal genes in Escherichia coli K-12 using PCR products. Proc Natl Acad Sci USA, 2000,97(12): p. 6640-5."; available for the public in the Institute of Bioengineering of the Academy of Military Medical Sciences of the Chinese People's Liberation Army. The replicons of the pKD46 plasmid are temperature sensitive, which would be disappeared during culture at 37°C, and this plasmid contains DNA encoding three recombinases for the Red recombination system, which is controlled by arabinose promoter.

pCP20 plasmid, disclosed in the literature "Datsenko, K.A. and B.L. Wanner, One-step inactivation of chromosomal genes in Escherichia coli K-12 using PCR products. Proc Natl Acad Sci USA, 2000,97(12): p. 6640-5."; available for the public in the Institute of Bioengineering of the Academy of Military Medical Sciences of the Chinese People's Liberation Army. The replicons of the pCP20 plasmid are temperature sensitive, which be lost during culture at 42°C. The plasmid contains DNA encoding FLP recombinase, which is controlled by a thermo-sensitive promoter, and the expression of FLP recombinase is induced at 42°C, while the plasmid is lost.

Aluminum hydroxide adjuvant Rehydragel LV was purchased from General Chemical Company.

### Example 1. Method for extending length of Salmonella paratyphi A O-antigen carbohydrate chain

### I. Knocking out cld gene encoding an enzyme controlling chain length of O-antigen of Salmonella paratyphi A CMCC50973 strain

### 1. Preparation of linear targeting DNA fragment 1

### 1) Design of PCR primer

The targeting fragment of the *cld* gene (sites 887812 to 888789) was designed according to the *S*. *paratyphi* ATCC9150 genome sequence (CP000026) published by GeneBank, and the *cld* gene of *Salmonella paratyphi A* CMCC50973 was knocked out. In the upstream and downstream of the *cld* gene, 500bp fragments were cut out and used as the homologous arms, and the targeting fragment of chloramphenicol resistant gene containing 500bp homologous sequence and FRT site at both ends was amplified by PCR. 50973*cld* up 5'and 50973*cld* down 3' were used to identify whether the *cld* gene was knocked out. The specific primer design was shown in Table 1.

**Table 1: Primers used for knocking out cld gene of Salmonella paratyphi A CMCC50973 strain**

| Name of primer | Primer sequence (5'→3') | Notes | Effect of primer pair |
|---|---|---|---|
| 50973 *cld* cat 5' | | Underlined part was *cld* homologous arm. | Amplifying to obtain chloramphenicol resistant gene fragments with *cld* upstream and downstream homologous arms. |
| 50973 *cld* cat 3' | | | |
| 50973 *cld* up 5' | CAGTTGCTGGCTAATTATCAGTCAGTGCCT(SEQ ID NO: 17) | | Amplifying *cld* primers of upstream and downstream homologous arms. |
| 50973 *cld* up 3' | GTCATAGATACCCTAACTAAAAAAAGGATGAAGC(SEQ ID NO: 18) | | |
| 50973 *cld* down 5' | TTCTTTGCCGGATGGTGGTCGGCTTCGAAA(SEQ ID NO: 19) | - | |
| 50973 *cld* down 3' | TTATGGACCAAAGGCGAAACCTCAGGCCAT(SEQ ID NO: 20) | | |

### 2) Acquisition of linear targeting DNA fragment 1

Using the plasmid pKD3 as template, primers 50973 *cld* cat 5'and 50973 *cld* cat 3' were used to amplify a chloramphenicol resistant gene fragment 50973 *cld* cat which had at both ends homologous arms 41bp upstream and downstream of the *cld* gene and FRT site; using *Salmonella paratyphi A* CMCC50973 genomic DNA as template, amplification was performed by using 50973 *cld* up5'and 50973 *cld* up3' to obtain *cld* upstream 500bp homologous arm 50973 *cld* up, while using 50973 *cld* down 5'and 50973 *cld* down 3' to obtained *cld* downstream 500bp homologous arm 50973 *cld* down, and then the 3 fragments 50973 *cld* cat, 50973 *cld* up and 50973 *cld* down were fused by PCR to obtain linear targeting DNA fragment 1 (SEQ ID NO: 11).

PCR reaction system was of 50µL, which comprised Q5 super-fidelity DNA polymerase 0.5µL, 5Xbuffer10µL, template 1µL, dNTP 4µL, primer 2.5µL, deionized water 29.5µL;

PCR reaction conditions: 98°C 20s; 98°C 10s, 55°C 10s, 72°C 50s, 30 cycles; 72°C 5min,to obtain the linear targeting DNA fragment 1 as shown in SEQ ID NO: 11. The product was separated by 1% agarose gel electrophoresis, the target strip was cut out from the gel, and the PCR product was recovered by using a DNA gel recovery kit. The procedure was carried out according to the instructions.

### 2. Acquisition of Salmonella paratyphi A (S.paratyphiCMCC50973Δcld) deficient in cld gene encoding an enzyme controlling chain length of O-antigen

### 1) Preparation of S. paratyphi CMCC50973/pKD46

*(1) S. paratyphi* CMCC50973 was inoculated in a LB liquid medium, incubated overnight at 37°C, transferred to LB liquid medium at 1: 100, and incubated at 37°C until OD₆₀₀ reached 0.6.
(2) Bacteria were collected by centrifugation, washed with autoclavation sterilized 10% glycerol (v/v) for four times, and finally were suspended with 400 µL of 10% glycerol, to obtain competent cells for electroporation, which were sub-packaged, ready to use.
(3) pKD46 plasmid was electroporated into the prepared *S. paratyphi* CMCC50973 competent cells, coated on a LB plates containing 100 µg/mL ampicillin, cultured at 30°C overnight, to obtain positive clones as *S. paratyphi* CMCC50973/pKD46 strain.

### 2) Acquisition of S. paratyphi CMCC50973 cld::cat strain

(1) The above obtained *S. paratyphi* CMCC50973/pKD46 was cultured overnight at 30 °C and passed at 1: 100 in a LB liquid medium containing ampicillin.
(2) When OD₆₀₀ was about 0.2,L-arabinose at a final concentration of 0.2% (w/v) was added to induce the expression of Red recombination system, and when OD₆₀₀ value was 0.6, *S. paratyphi* CMCC50973/pKD46 competent cells were prepared by the steps as described above.
(3) 10 µL of the linear targeting DNA fragment 1 obtained in the above Step 1 was taken and electroporated into the sub-packaged *S. paratyphi* CMCC50973/pKD46 competent cells.
(4) The cells were rapidly added to 1 mL of pre-cooled LB medium, resuscitated at 30 °C for about 2.5 h, and then coated on a LB plate containing 30 µg/mL chloramphenicol at a final concentration, and placed in a 30 °C incubator for culture overnight.
(5) The positive clones were screened, and subjected to PCR identification using primers 50973 *cld* cat 5'and 50973 *cld* cat 3', in which the wild-type bacteria could not be amplified to generate fragments, the *cld* gene was identified to have a size of 1100bp after being substituted by *cat* gene. According to the PCR results, positive clones of *S. Paratyphi* CMCC50973 cells in which the *cld* gene was replaced by the *cat* gene were screened out, to obtain the deletion mutant *S. paratyphi* CMCC50973 *cld::cat*/pKD46.

### 3) Acquisition of S. paratyphi CMCC50973Δcldstrain

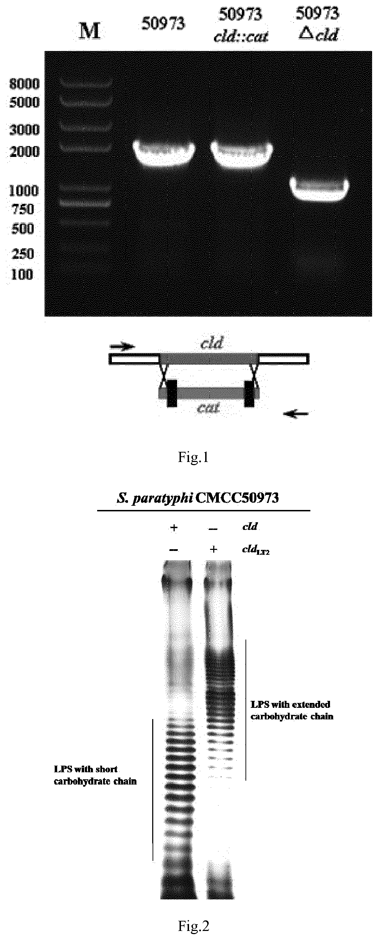

(1) The positive clone *S. paratyphi* CMCC50973 *cld::cat*/pKD46 was inoculated into a LB liquid medium containing 30 µg/mL chloramphenicol, cultured and passed at 37 °C for three times (cultured for 12 h each time), to remove pKD46 plasmid, so as to obtain *S. paratyphi* CMCC50973 *cld::cat* strain.
(2) The *S. paratyphi* CMCC50973 *cld::cat* as prepared according to the above method was used for electroporation of the competent cells, and plasmid pCP20 was electroporated into *S. paratyphi* CMCC50973 *cld::cat* competent cells, and then coated with a LB plate containing ampicillin, so as to obtain *S. paratyphi* CMCC50973 *cld::cat*/pCP20 strain.
(3) *S. paratyphi* CMCC50973 *cld::cat*/pCP20 monoclones were picked and placed in a LB liquid medium, incubated at 30 °C until OD₆₀₀ was about 0.6, and transferred and cultured at 42 °C overnight.
(4) The bacterial solution cultured overnight at 42 °C was streaked on a LB plate, and monoclones were picked out on LB plates and LB plates containing chloramphenicol. The monoclones that grew on the LB plates but did not grow on chloramphenicol-containing LB plates were chosen, and subjected to PCR identification by using primers 50973 *cld* up 5'and 50973 *cld* down 3'. The PCR identification showed the size of wild *S. paratyphi* CMCC50973 fragment was 2028bp, while the size after knockout of the *cld* gene was 1100 bp. The results of the PCR identification were shown in Fig.1. The positive clone was named *S. paratyphi* CMCC50973Δcld.

### II. Acquisition and phenotype identification of O-antigen carbohydrate chain-extended Salmonella paratyphi A

### 1. Construction of a vector expressing cld_{LT2} gene encoding an enzyme controlling chain length of O-antigen of Salmonella typhimurium

The DNA sequence as shown in SEQ ID NO: 1 was synthesized according to the nucleotide sequence of *cld* gene (GenBank No.: NC_003197.1, position 2156832 to 2157815) encoding an enzyme controlling chain length of O-antigen of *Salmonella typhimurium,* and this gene was named as *cld*_{LT2}. The enzyme controlling chain length of O-antigen has an amino acid sequence as shown in SEQ ID NO: 2, and the *cld*_{LT2} gene encoding the enzyme has a sequence as shown in SEQ ID NO: 1.

The synthesized *cld*_{LT2} gene was digested with EcoRI and Hind III, linked into pMMB66EH expression vector (purchased from ATCC, ATCC37620), to construct transition vector pMMB66EH-*cld*_{LT2}, the primers 66tac 5' and 66tac 3' were designed by using pMMB66EH-*cld*_{LT2} as template, the expression cassette carrying *cld_{LT2}* gene was amplified, and linked to pET28a(purchased from Novagen) between restriction enzyme cutting sites of Xba I and XhoI, so as to construct expression vector pETtac28-*cld*_{LT2}. The primers were as follows:
66tac 5': AAAATCTAGAGCGCCGACATCATAACGGTTCTGGCA (SEQ ID NO: 21);
66tac 3': TTTTCTCGAGCGTTCACCGACAAACAACAGATAA (SEQ ID NO: 22).

The sequencing results showed that the sequence as shown in SEQ ID NO: 1 was inserted between the restriction enzyme cutting sites of Xba I and Xho I in the pET28a vector, which indicated the vector was correct.

### 2. Salmonella paratyphi A LPS length detection

The above-prepared *S. paratyphi* CMCC50973Δcld electroporation competent cells were electroporated with the above-obtained pETtac28-*cld*_{LT2}, coated on kanamycin-containing LB plates, and positive clones were picked and cultured at 37 °C. When OD₆₀₀ was about 0.6, IPTG with a final concentration of 1 mM was added to induce expression of *cld*_{LT2} gene.

After 10 h of induction, 1 mL of culture was taken and the cells were collected by centrifugation, washed with PBS once, and 100 µL of lysis buffer (2 mL of 10% SDS, 0.4 mL of 2-mercaptoethanol, 1 mL of 100% glycerol, 5mL of 2M Tris-HCL pH 6.8, 20µL of 10% bromophenol blue, 1.6mL of ddH₂O) was added and fully mixed, heated with boiling water for 10min, added with 4µL of 20mg/mL of proteinase K, reacted at 60 °C for 1-2h, 15µL of product was taken for loading and performing SDS- PAGE, in which the separation gel was 15%, the concentrated gel was 4%, and the electrophoresis was ceased after the bromophenol blue gel exuded for 30 minutes.

The above-mentioned polyacrylamide gel was placed in an immobilized solution overnight, added with a sensitizing solution and reacted for 30min, washed with deionized water 3 times, 15min per time, added a silver nitrate solution and reacted for 20min, washed twice with deionized water, 1min per time, add with a developer solution for color development, the reaction was finally terminated, and the product was washed with deionized water. At the same time, the wild strain of *S*. *paratyphi* CMCC50973 was used as control.

The results were showed in Fig.2, in which the result of silver staining test showed that the LPS of the wild *S. paratyphi* CMCC50973 was distributed in the lower molecular weight region, while when the *cld* gene thereof was replaced by *cld*_{LT2}, relatively concentrated LPS stripes appeared in the higher molecular weight region, which indicated that by replacing the *cld* gene of *S. paratyphi* CMCC50973 with *cld*_{LT2}, the O-antigen carbohydrate chain thereof was significantly extended.

### Example 2. Salmonella paratyphi A O-antigen-recombinant fusion protein conjugate vaccine as prepared by one-step bio-crosslinking method and uses thereof

### I. Construction of O-antigen ligase gene waaL deficient Salmonella paratyphi A

### (I) Preparation of linear targeting DNA fragment 2

### 1. Design and synthesis of PCR primers

By referring to the *waaL* gene (GeneBank No.CP000026, sites 3696236-3697450) in the whole genome sequence (NC_006511) of the *Salmonella paratyphi A* 50973 strain (*S. paratyphi* CMCC50973) and its upstream and downstream sequences, a pair of primers was designed for each of upstream (5' end) and downstream (3' end) of the *waaL* gene, namely 73waaLu1/73waaLu2 and 73waaLd1/73waaLd2. For ease of manipulation, the restriction sites *BamH* I and Sa*l* I were added to the primer ends of the upstream homologous arm up, and the restriction sites *Hind* III and *Xho* I were added to the primer ends of the downstream homologous arm down. In the meantime, besides the *waaL* gene upstream and downstream homologous arms on the genome, a pair of primers (73waaLw1/73waaLw2), a pair of internal detection primers (73waaLn1/73waaLn2) and a pair of *kan* gene primers (Kan1/Kan2) were designed for simultaneous sequencing and verification, in order to test whether the mutants were constructed successfully. The above-mentioned primers had sequences as shown in Table 2 (the underlined sequences were recognition sites for restriction enzyme digestion).

**Table 2. Sequences of primers**

| Primer | Sequence (5'→3') | Use |
|---|---|---|
| 73waaLu1 | CGGGATCCAGGCTTTGACTATGTGGA (SEQ ID NO: 23) | Amplifying up fragment |
| 73waaLu2 | GCGTCGACATCTGGCGATATGAGTATG(SEQ ID NO: 24) | |
| 73waaLd1 | CCAAGCTTTAGTGCAGGCATATTGGG (SEQ ID NO: 25) | Amplifying down fragment |
| 73waaLd2 | CCCTCGAGTATCACCTCGCAGAACCT (SEQ ID NO: 26) | |
| 73waaLw1 | AACACCGGATTACGGATAA (SEQ ID NO: 27) | Identifying mutants |
| 73waaLw2 | TGCATGGTGGCTGTAGAA (SEQ ID NO: 28) | |
| 73waaLn1 | AGAAACGGTTGCGAAAAT (SEQ ID NO: 29) | Identifying mutants |
| 73waaLn2 | ATAGCCGTAGCCCTTGAT (SEQ ID NO: 30) | |
| Kan1 | GCGTCGACGTGTAGGCTGGAGCTGCTTC(SEQ ID NO: 31) | Identifying mutants |
| Kan2 | CCAAGCTTATGGGAATTAGCCATGGTCC(SEQ ID NO: 32) | |

### 2. Construction of linear targeting DNA fragment 2

1) Using the genomic DNA of *Salmonella paratyphi A* 50973 as template, the upstream and downstream homologous arms, up fragment and down fragment, of the *waaL* gene were amplified by 73waaLu1/73waaLu2 and 73waaLd1/73waaLd2, respectively. The PCR procedures were as follows: 94 °C 10min; 94 °C 30s, 50 °C 30s, 72 °C 30s, 30 cycles; 72 °C 10min.
2) Construction of pETKan
   The *kan* gene fragment represented by the nucleotide sequence at sites 578-2073 in SEQ ID NO: 12 was artificially synthesized, and the *kan* gene fragment and pET-22b plasmid were digested with the restriction enzymes *Sal*I and *Hind* IIII, and a recombinant plasmid was obtained after ligating, and named as pETKan. The pETKan was sequenced and the results were correct.
3) The up fragment was doubly digested with restriction endonucleases *BamH* I and *Sal*I to obtain a gene fragment 1; the plasmid pETKan was doubly digested with restriction endonucleases *Bam*H I and *Sal* I to obtain a vector large fragment 1; and the gene fragment 1 was ligated with the vector large fragment 1 to obtain an intermediate vector 1;
   the down fragment was doubly digested with restriction endonuclease *Hind* III and *Xho* I to obtain a gene fragment 2; the intermediate vector 1 was doubly digested with restriction endonuclease *Hind* III and *Xho* I to obtain a vector large fragment 2; and the gene fragment 2 was ligated with the vector large fragment 2 to obtain an intermediate vector 2.
4) The intermediate vector 2 was doubly digested with restriction endonucleases *BamH* I and *Xho* I to obtain the desired targeting DNA fragment that had homologous arms at both sides and contained the *kan* gene in the middle part, and the fragment has a nucleotide sequence as shown in SEQ ID NO: 12. In the SEQ ID NO: 12, as counting from the 5' end, the nucleotides at sites 7-571 were the up fragment, the nucleotides at sites 578-2073 were the *kan* gene, and the nucleotides at sites 2080-2543 were the down fragment.

A linear targeting DNA fragment 2 (SEQ ID NO: 12) with a concentration of up to 300 ng/µL was obtained by further PCR amplification of the target fragment by using the DNA fragment shown in SEQ ID NO: 12 as template and 73waaLu1 and 73waaLd2 as primers.

### (II) Construction of S. paratyphi CMCC50973/pKOBEG

Since the pKOBEG plasmid contained the various enzymes required to encode the λ-Red recombination system, the pKOBEG plasmid was electroporated into *S. paratyphi* CMCC50973 competent cells, coated to chloramphenicol resistant (pKOBEG plasmid resistance, chloramphenicol) LB plate, and cultured at 30 °C overnight to obtain a positive clone, which was named as *S. paratyphi* CMCC50973/pKOBEG strain.

### (III) Using linear targeting DNA fragment 2 to electroporate S. paratyphi CMCC50973/pKOBEG

1. *S. paratyphi* CMCC50973/pKOBEG was inoculated to a low salt LB medium containing chloramphenicol in a final concentration of 30 µg/mL and cultured overnight at 30 °C, then passaged to a low salt LB liquid medium at a volume ratio of 1:100, and continuously cultured.
2. The culture medium in Step 1 was added with L-arabinose at a final concentration of 1 mmol/L at 1 hour before the OD₆₀₀ value reached 0.6, so as to induce the expression of the Red recombination system.
3. When the OD₆₀₀ value of the culture medium in step 2 reached 0.6, 5 µL of the 300 ng/µL linear targeting DNA fragment 2 as prepared in step (I) was taken and used to electroporate and transform *S. paratyphi* CMCC50973/pKOBEG.
4. 1 mL of pre-cooled low salt LB liquid medium was rapidly added to the transformed cells, resuscitation was performed at 30 °C for about 2.5 hours, and then the medium was coated on LB plates containing kanamycin at a concentration of 50 µg/mL, placed in 30 °C incubator and cultured overnight, and positive clones were screened out.
5. The positive clones were inoculated into a liquid LB medium (with kanamycin resistance at a concentration of 50 µg/mL), cultured and passaged twice at 42°C (12 hours each time) to remove pKOBEG plasmid, and finally obtain a mutant with kanamycin resistance *waaL* deletion, named *S*. *paratyphi* CMCC50973 *waaL::kan*.

(IV) The plasmid pCP20 coding FRT site-specific recombinase was electroporated and transferred into *S. paratyphi* CMCC50973*waaL::kan*, cultured at 30 °C on a LB plate that contained chloramphenicol at a concentration of 50µg/mL and was free of kanamycin, positive clones of Cm^{r}Km^{s} (chloramphenicol resistant, Kanamycin sensitive) were screened out.

(V) The positive clones screened out in step (IV) were transferred into a liquid LB and cultured at 42 °C for 12 h to obtain a mutant with deletion of target genes that did not contain kanamycin and plasmid pCP20, which were named as *S. paratyphi* CMCC50973ΔwaaL, so that *Salmonella paratyphi A* with deletion of *waaL* gene was obtained.

### II. Construction of Salmonella paratyphi A with deletion of waaL gene and cld gene

### (I) Preparation of linear targeting DNA fragment 2

The steps were the same of the above step (I).

### (II) Construction of S. paratyphi CMCC50973Δcld/pKOBEG

Since the pKOBEG plasmid contained the various enzymes required to encode the λ-Red recombination system, the pKOBEG plasmid was electroporated and transformed into *S. paratyphi* CMCC50973Δcld competent cells, and coated a LB plate with chloramphenicol resistance (pKOBEG plasmid resistance, chloramphenicol), cultured at 30 °C overnight to give a positive clone named *S*. *paratyphi* CMCC50973Δ*cld*/pKOBEG strain.

### (III) Using linear targeting DNA fragment 2 to electroporate S. paratyphi CMCC50973Δcld/pKOBEG

1. *S. paratyphi* CMCC50973Δ*cld*/pKOBEG was inoculated to a low salt LB liquid culture medium containing chloramphenicol at a final concentration of 30µg/mL and cultured at30°C overnight, and then passaged to a low salt LB liquid medium at a volume ratio of 1:100, and continuously cultured.
2. The culture medium in Step 1 was added with L-arabinose at a final concentration of 1 mmol/L at 1 hour before the OD₆₀₀ value reached 0.6, so as to induce the expression of the Red recombination system.
3. When the OD₆₀₀ value of the culture medium in step 2 reached 0.6, 5 µL of the 300 ng/µL linear targeting DNA fragment 2 as prepared in step (I) was taken and used to electroporate and transform *S. paratyphi* CMCC50973Δ*cld*/pKOBEG.
4. 1 mL of pre-cooled low salt LB liquid medium was rapidly added to the transformed cells, resuscitation was performed at 30 °C for about 2.5 hours, and then the medium was coated on LB plates containing kanamycin at a concentration of 50 µg/mL, placed in 30 °C incubator and cultured overnight, and positive clones were screened out.
5. The positive clones were inoculated into a liquid LB medium (with kanamycin resistance at a concentration of 50 µg/mL), cultured and passaged twice at 42°C (12 hours each time) to remove pKOBEG plasmid, and finally obtain a mutant with kanamycin resistance *waaL* deletion, named *S*. *paratyphi* CMCC50973Δ*cldwaaL::kan*.

(IV) The plasmid pCP20 coding FRT site-specific recombinase was electroporated and transferred into *S. paratyphi* CMCC50973Δ*cldwaaL::kan*, cultured at 30 °C on a LB plate that contained chloramphenicol at a concentration of 50µg/mL and was free of kanamycin, positive clones of Cm^{r}Km^{s} (chloramphenicol resistant, Kanamycin sensitive) were screened out.

(V) The positive clones screened out in step (IV) were transferred into a liquid LB and cultured at 42 °C for 12 h to obtain a mutant with deletion of target genes that did not contain kanamycin and plasmid pCP20, which were named as *S. paratyphi* CMCC50973Δ*cld*Δ*waaL.*

### II. Molecular identification of S. paratyphi CMCC50973ΔcldΔwaaL

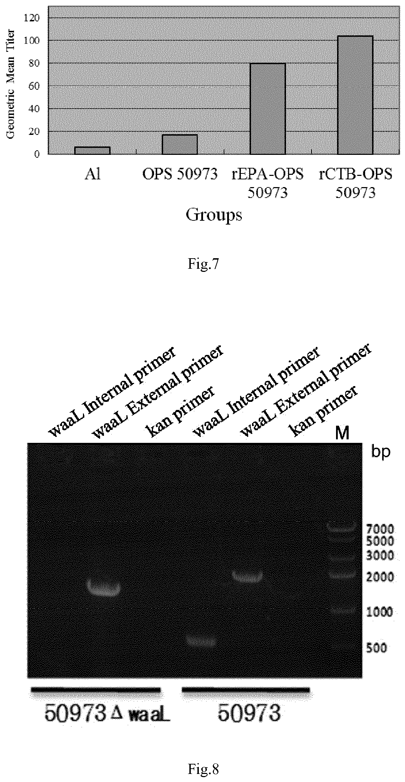

The genomic DNAs of *S. paratyphi* CMCC50973Δcld and *S. paratyphi* CMCC50973ΔcldΔwaaL were separately used as templates, and PCR identification was performed by separately using a pair of *waaL* internal primers (73waaLn1/73waaLn2), a pair of *waaL* external primers (73waaLw1/73waaLw2) and kan primers (kan1/kan2). The results are shown in Fig.8; and in Fig.8, 50973Δ*waaL* represents *S. paratyphi* CMCC50973Δ*cld*Δ*waaL*; 50973 represents *S. paratyphi* CMCC50973Δ*cld*.

The results showed that there was not a target strip when the PCR amplification was performed by using the genomic DNA of *S. paratyphi* CMCC50973Δ*cld*Δ*waaL* as template and *waaL* internal primers as primers; while there was a target strip when the PCR amplification was performed by using the genomic DNA of *S. paratyphi* CMCC50973Δ*cld* as template and *waaL* internal primers as primers. Moreover, since the *S. paratyphi* CMCC50973Δ*cld*Δ*waaL* had knockout of *waaL* gene, the target strip obtained when performing the PCR amplification by using the genomic DNA of *S. paratyphi* CMCC50973Δ*cld*Δ*waaL* as template and *waaL* external primers as primers was smaller than the strip obtained when performing PCR amplification by using the genomic DNA of *S. paratyphi* CMCC50973Δ*cld* as template and *waaL* external primers as primers. As a result of the removal of the *Kan* resistance gene, there was not a target strip when the PCR amplification was performed by using the genomic DNA of *S. paratyphi* CMCC50973Δ*cld*Δ*waaL* as a template and the *kan* primer as a primer.

These results demonstrated the successful construction of the *Salmonella paratyphi* A 50973 mutant *S. paratyphi* CMCC50973Δ*cld*Δ*waaL*, which had *waaL* gene deletion and *cld* gene deletion.

### III. Construction of glycosylation engineering Salmonella paratyphi A

### 1. Construction of rEPA4573 and rCTB4573 expression vectors

A recombinant *Pseudomonas aeruginosa* exotoxin A fusion protein (rEPA4573) was constructed according to the amino acid sequence of *Pseudomonas aeruginosa* exotoxin A (AE004091.2) published by GeneBank, in which its signal peptide (the first 25 amino acids) was replaced by DsbA signal peptide, its E at position 553 was deleted, in the meantime, the L at position 552 was mutated as V, and its C-terminal was fused with the polypeptide sequences shown in positions 45-73 amino acids (defined as Pla) of *Neisseria meningitidis* pilin PiLE (NC_003112.2) and 6×Histag. The amino acid sequence of the optimized rEPA4573 was shown in SEQ ID NO: 4, wherein the 1-19 positions were the amino acid sequence of the DsbA signal peptide; the amino acids at positions 20-631 were the amino acid sequence of non-toxic mutant of *Pseudomonas aeruginosa* toxin protein A; the amino acids 637-665 were the amino acid sequence of the polypeptide at 45-73 positions of *Neisseria meningitidis* pilin PiLE (NC_003112.2), and the amino acids 666-674 were flexible linker sequence and 6×His tag sequence; the optimized gene sequence of rEPA4573 was shown in SEQ ID NO: 3. The artificially synthesized rEPA4573 coding sequence was digested with EcoR I and Hind III, and ligated into pMMB66EH expression vector (ATCC, ATCC37620) to construct pMMB66EH-rEPA4573 vector.

Sequencing results showed that the sequence shown in SEQ ID NO: 3 was inserted between the EcoR I and Hind III cleavage sites of the pMMB66EH expression vector, indicating that the vector was correct.

The recombinant CTB fusion protein (rCTB4573) was constructed according to the amino acid sequence of cholera toxin B subunit (CTB) (X76390.1) published by GeneBank, in which its signal peptide (the first 21 amino acids) was replaced by the DsbA signal peptide, and the C-terminal of the recombinant fusion protein was fused with the polypeptide sequence at the 45-73 positions of *Neisseria meningitidis* pilin PiLE (NC_003112.2) and 6×His tag. The amino acid sequence of the optimized recombinant CTB fusion protein was set forth in SEQ ID NO: 6, wherein the 1-19 positions were the amino acid sequence of the DsbA signal peptide, the positions 20-122 were the amino acid sequence of the cholera toxin B subunit, the 128-156 positions were the amino acid sequence at the 45-73 position of *Neisseria meningitidis* PilE (NC_003112.2), the 157-166 positions were the flexible linker and the6×His tag sequence; the coding sequence of the optimized recombinant CTB fusion protein was shown in SEQ ID NO: 5. The artificially synthesized gene encoding recombinant CTB fusion protein was digested with EcoR I and Hind III, and ligated into pMMB66EH expression vector (ATCC, ATCC37620) to construct pMMB66EH-rCTB4573 vector.

Sequencing results showed that the sequence shown in SEQ ID NO: 5 was inserted between the EcoR I and Hind III restriction sites of the pMMB66EH expression vector, indicating that the vector was correct.

### 2. Construction of cld_{LT2} and pglL tandem expression vectors

According to the amino acid sequence of the *Neisseria meningitidis* O-oligosaccharide transferase PglL (JN200826.1) published by GeneBank, its DNA sequence was synthesized by whole gene synthesis technique. The amino acid sequence of *Neisseria meningitidis* O-oligosaccharide transferase PglL was shown in SEQ ID NO: 8, and the gene encoding *Neisseria meningitidis* O-oligosaccharide transferase PglL was shown in SEQ ID NO: 7.

The artificially synthetized gene encoding PglL was digested with EcoR I and Hind III, ligated into pKK223-3 vector (commercially available from Uppasla Pharmacia LKB Biotechniligy AB, Sweden), and primers 223tac-box5'and 223tac-box3' were used for amplification to obtain the expression cassette of PglL, and ligated to the Bgl II site of pETtac28-*cld*_{LT2} obtained in Example 1, so as to construct the pETtac28-*pglL*-*cld*_{LT2} recombinant expression vector. The primer sequences are as follows:
223tac-box5': ATCGAGATCTACTGCATAATTCGTGTCGCTCAAG (SEQ ID NO: 33);
223tac-box3': ATCGAGATCTGTCTCATGAGCGGATACATATTTG (SEQ ID NO: 34).

### 3. Construction of pglL expression vector

The expression cassette of *pglL* prepared in the above step 2 was ligated to the Bgl II site of pET28a (commercially available from Novagen) to construct a pETtac28-*pglL* recombinant expression vector.

### 4. Construction of S. paratyphi CMCC50973ΔcldΔwaaL/pMMB66EH-rEPA4573/pETtac28-pglL-cld_{LT2}

The pMMB66EH-rEPA4573 and pETtac28-*pglL*-*cld*_{LT2} plasmids were electroporated orderly into the *S. paratyphi* CMCC50973Δ*cld*Δ*waaL*electroporation competent cells as prepared by the above methods, so as to construct the glycosylation engineering *Salmonella paratyphi A S. paratyphi* CMCC50973Δ*cld*Δ*waaL*/pMMB66EH-rEPA4573/pETtac28-*pglL*-*cld*_{LT2}.

### 5. Construction of S. paratyphi CMCC50973ΔwaaL/pMMB66EH-rEPA4573/pETtac28-pglL

The pMMB66EH-rEPA4573 and pETtac28-*pglL*plasmids were electroporated orderly into the *S*. *paratyphi* CMCC50973Δ*waaL* electroporation competent cells as prepared by the above methods, so as to construct the glycosylation engineering *Salmonella paratyphi A S. paratyphi* CMCC50973Δ*waaL*/pMMB66EH-rEPA4573/pETtac28-*pglL*.

### 6. Construction of S. paratyphi CMCC50973ΔcldΔwaaL/pMMB66EH-rCTB4573/pETtac28-pglL-cld_{LT2}

The pMMB66EH-rCTB4573 and pETtac28-*pglL*-*cld*_{LT2} plasmids were electroporated orderly into the *S. paratyphi* CMCC50973Δ*cld*Δ*waaL* electroporation competent cells as prepared by the above methods, so as to construct the glycosylation engineering *Salmonella paratyphi A S. paratyphi* CMCC50973Δ*cld*Δ*waaL*/pMMB66EH-rCTB4573/pETtac28-*pglL*-*cld*_{LT2}.

### 7. Construction of S. paratyphi CMCC50973ΔwaaL/pMMB66EH-rCTB4573/pETtac28-pglL

The pMMB66EH-rCTB4573 and pETtac28-*pglL*plasmids were electroporated orderly into the *S*. *paratyphi* CMCC50973Δ*waaL*electroporation competent cells as prepared by the above methods, so as to construct the glycosylation engineering *Salmonella paratyphi A S. paratyphi* CMCC50973Δ*waaL*/pMMB66EH-rCTB4573/pETtac28-*pglL*.

### 8. Construction of S. paratyphi CMCC50973ΔwaaL/pMMB66EH-rCTB4573

The pMMB66EH-rCTB4573 plasmid was electroporated orderly into the *S. paratyphi* CMCC50973Δ*waaL* electroporation competent cells as prepared by the above methods, so as to construct the glycosylation engineering *Salmonella paratyphi A S. paratyphi* CMCC50973Δ*waaL*/pMMB66EH-rCTB4573.

### 9. Construction of S. paratyphi CMCC50973ΔwaaL/pMMB66EH-rEPA4573

The pMMB66EH-rEPA4573plasmid was electroporated orderly into the *S. paratyphi* CMCC50973Δ*waaL*electroporation competent cells as prepared by the above methods, so as to construct the glycosylation engineering *Salmonella paratyphi A S. paratyphi* CMCC50973Δ*waaL*/pMMB66EH-rEPA4573.

### IV. Detection of glycosylation situation of rEPA4573 and rCTB4573

A single clone of glycosylation engineering bacteria was picked and inoculated into a LB medium containing ampicillin at a final concentration of 100 µg/mL and kanamycin at a final concentration of 50 µg/mL, cultured at 37 °C until OD₆₀₀ was about 0.6, and then IPTG was added at a final concentration of 1 mM, cooled to perform induction at 16 °C for 20 h.

On the next day, 1mL of the bacterial liquor induced at 16 °C for 20 h was taken, centrifuged to take the bacteria, the bacteria were slowly suspended with IX reduction buffer, treated with boiling water bath for 10min, then subjected to SDS-PAGE electrophoresis. After electrophoresis was completed, the protein was transferred to a PVDF membrane by a Bio-Lab semi-dry transfer, the transfer was performed at constant voltage of 20V for 1 h, and anti-His mouse monoclonal antibody (commercially available from Sigma, Cat. A7058) was used for detection, in which specific procedures could be seen in the Molecular Cloning Guide.

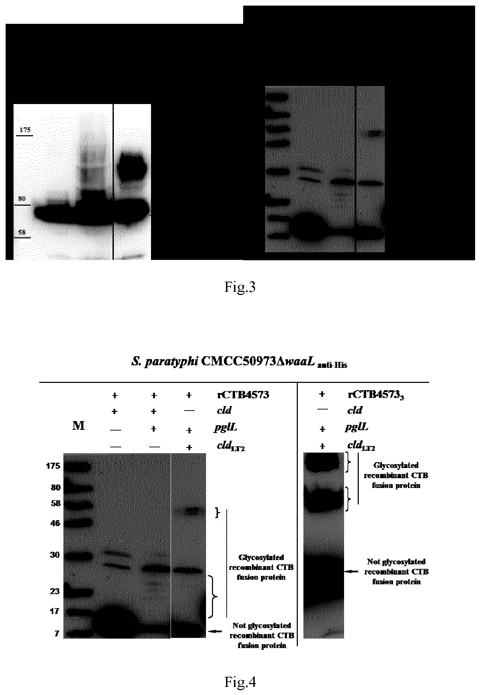

It can be seen from Fig.3 that the molecular weights of the glycosylation-modified rEPA4573-OPS_{Spty50973} and rCTB4573-OPS_{Spty50973} were significantly increased after the *cld* gene of *S. paratyphi* CMCC50973 itself was replaced with *cld*_{LT2}, indicating that after the substitution of *cld*_{LT2}, the polysaccharide protein ratio of glycoprotein was significantly improved.

### V. Increasing the proportion of polysaccharides in Salmonella paratyphi A O-polysaccharide-recombinant CTB fusion protein via tandem O-glycosylation sites

### 1. Construction of recombinant CTB fusion protein (rCTB4573₃) expression vector containing three P1a sequences

In order to increase polysaccharide-protein ratio of glycoprotein, the present invention performed tandem fusion at C-terminus of CTB with 3 polypeptide sequences (P1a sequence) of the positions 45-73 of *Neisseria meningitidis* pilin PilE (NC_003112.2), i.e., rCTB4573₃ The amino acid sequence of the recombinant CTB fusion protein was shown in SEQ ID NO: 10, wherein the positions 1-19 were the amino acid sequence of the DsbA signal peptide; the positions 20-122 were the amino acid sequence of the cholera toxin B subunit; the positions 123-127 were the flexible linker; the positions 128-222 were the amino acid sequence of the 3 polypeptide sequences of the positions 45-73 of *Neisseria meningitidis* pilin PilE (NC_003112.2); the positions 223-232 were 6×His tag sequence; the coding sequence of the recombinant CTB fusion protein was shown in SEQ ID NO: 9. Wherein, the gene encoding the polypeptide as set forth in positions 45 to 73 of *Neisseria meningitidis* pilin PilE (NC _003112.2) was shown in SEQ ID NO: 14, and the protein sequence encoded by this gene was shown in SEQ ID NO: 13.

The artificially synthesized coding sequence of the recombinant CTB fusion protein was digested with EcoR I and Hind III, and ligated into pMMB66EH expression vector (ATCC, ATCC37620) to construct pMMB66EH-rCTB4573₃ vector.

The sequencing results showed that the sequence shown in SEQ ID NO: 9 was inserted between the EcoR I and Hind III restriction sites of the pMMB66EH expression vector, indicating that the vector was correct.

### 2. Construction of S. paratyphi CMCC50973ΔcldΔwaaL/pMMB66EH-rCTB4573₃/pETtac28-pglL-cld_{LT2}

The pMMB66EH-rCTB4573₃ and pETtac28-*pglL*-*cld*_{LT2} plasmids were electroporated into the *S*. *paratyphi* CMCC50973Δ*cld*Δ*waaL*electroporation competent cells as prepared by the above methods, so as to construct the glycosylation engineering *S*. *paratyphi* CMCC50973Δ*cld*Δ*waaL*/pMMB66EH-rCTB4573₃/pETtac28-*pglL*-*cld*_{LT2}.

### 3. Preparation of rCTB4573₃-OPS_{Spty50973}

The method was the same as above mentioned.

It can be seen from Fig.4 that after the addition of three glycosylation sites, the recombinant CTB fusion protein underwent O-glycosylation modification, and at the same time, due to the addition of multiple glycosylation sites, the existence of multiple clusters of glycosylation strips indicated the multiple sites were all effective glycosylation sites.

### VI. Acquisition of rEPA4573-OPS_{Spty50973} and rCTB4573₃-OPS_{Spty50973}

### 1. Purification ofrEPA4573-OPS_{Spty50973}

Monoclones of the glycosylation engineering strain *S*. *paratyphi* CMCC50973Δ*cld*Δ*waaL*/pMMB66EH-rEPA4573/pETtac28-*pglL*-*cld*_{LT2} were picked out, inoculated to a LB culture plate containing ampicillin and kanamycin double resistances, cultured at 37 °C until OD₆₀₀ reached about 0.6, then IPTG at a final concentration of 1mM was added, and cooled to perform induction at 25 °C for 20h.

### 1) Pre-treatment of samples

10g of the above bacteria after induction at 25 °C for 20hwas taken, added with 100 mL of purified water, subjected to ultrasonication (ultrasonication for 3s and suspension for 5s, cumulative ultrasonication time 30 min), centrifuged at 12000g of centrifugal force, and the supernatant was collected. To this crude extract, pH 7.5 Tris-HCL at a final concentration of 20mM, NaCL at a final concentration of 0.2M and imidazole at a final concentration of 10mM were added, fully stirred, then centrifuged again at 12000g of centrifugal force, and the supernatant was collected and used as a crude extract containing the recombinant EPA fusion protein (rEPA4573-OPS*_{Spty50973}*) that was modified by *Salmonella paratyphi A* OPS.

### 2) Purifying samples with Chelating affinity chromatographic column

Chelating affinity chromatographic column (Φ1.6cm^{∗}15cm) was used for primary purification of samples. The column bed was first rinsed with 3 column bed volumes of 0.5M NaOH, then equilibrated with deionized water to neutral pH, then equilibrated with 3 column bed volumes of 0.5M NiSO₄, and then equilibrated with 1 column bed volume of B1 solution (20mM pH 7.5 Tris-HCl, 0.5M NaCl, 500mM imidazole), and finally equilibrated with 3 column bed volumes of A1 solution (20mM pH 7.5 Tris-HCl, 0.5M NaCl, 10mM imidazole), wherein the above-used flow rate was always 4 mL/min. The above crude extract containing rEPA4573-OPS_{*Spty*50096} was loaded from A tube, and the unbound protein was washed off with solution A, and the elution was finally performed by using 100% B1 to collect 30 mL of eluate.

### 3) Desalting samples

The sample that was preliminarily purified by the Chelating affinity chromatographic column was desalted by using G25 fine chromatographic column (Φ1.6cm ^{∗} 30cm), in which the mobile phase was A3 solution (20mM pH 7.5 Tris-HCl). The column bed was firstly rinsed with3 column bed volumes of 0.5M NaOH, then equilibrated with deionized water to pH neutral, and finally equilibrated with 3 column volumes of A3 solution. The sample was loaded from A tube, 60 mL of sample was collected, and the above-used flow rate was always 4 mL/min.

### 4) Further purification of rEPA4573-OPS_{Spty50973} by using ProteinPak DEAE8HR anion exchange chromatographic column

The desalted sample was further purified by ProteinPak DEAE8HR anion exchange chromatographic column (waters).The column bed was first rinsed with 3 column bed volumes of 0.5M NaOH, then equilibrated with deionized water to pH neutral, and then equilibrated with 3 column bed volumes of A3 solution (20mM pH 7.5 Tris-HCl). The sample was loaded from A tube, the unbound glycoprotein was washed off with A3 solution, then linear elution was performed using 0-50% B3 solution (20mM pH 7.5 Tris-HCl, 1M NaCl) for 30 min, and the eluate was collected, in which the above used flow rate was always 1 mL/min. The peak position of glycoprotein rEPA4573-OPS_{*Spty*50973} was at position of about 8-18 mS/cm.

### 5) Fine purification of rEPA4573-OPS_{Spty50973} by using Superdex 75 chromatographic column

The sample as purified by ProteinPak DEAE8HR anion exchange chromatographic column was further purified by using Superdex 75 FPLC (Φ1cm ^{∗} 30cm, GE). The column bed was first washed with 3 column bed volumes of 0.5M NaOH, then equilibrated with deionized water to pH neutral, and then equilibrated with 3column bed volumes of A4 solution (20mM pH 7.5 PB, 0.9% NaCl). The sample in volume of 1mL was loaded from a sample loop, and 8 to 11 mL of the effluent sample was collected, and this sample was the purified rEPA4573-OPS_{*Spty*50973}.

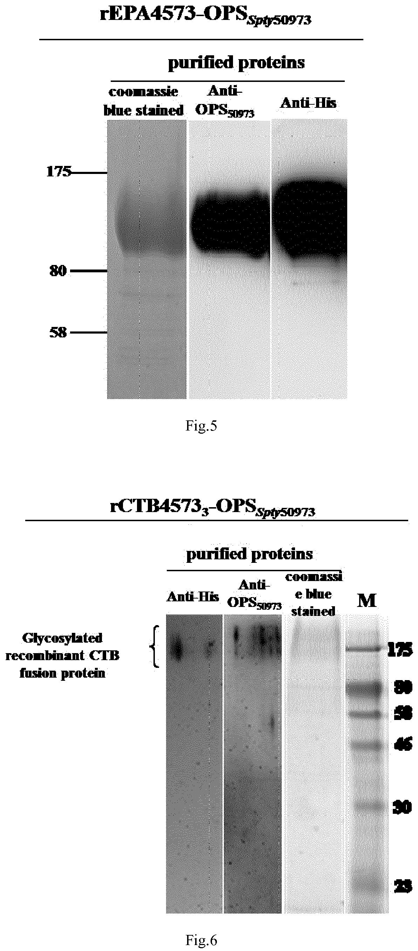

This sample was analyzed by 8% SDS-PAGE and western blot, and the results were shown in Fig.5.

### 2. Purification of rCTB4573₃-OPS_{Spty50973}

Monoclone of the glycosylation engineering strain *S*. *paratyphi* CMCC50973Δ*cld*Δ*waaL*/pMMB66EH-rCTB4573₃/pETtac28-*pglL*-*cld*_{LT2} was picked out, inoculated to a LB culture medium with ampicillin and kanamycin double resistance, cultured at 37°C until OD₆₀₀was about0.6, then IPTG at a final concentration of 1mM was added, and cooled to perform induction at 16°C for 20h.

### 1) Pre-treatment of samples

10g of the above bacteria after induction at 16 °C for 20h was taken, added with 100 mL of purified water, subjected to ultrasonication (ultrasonication for 3s and suspension for 5s, cumulative ultrasonication time 30 min), centrifuged at 12000g of centrifugal force, and the supernatant was collected. To this crude extract, pH 7.5 Tris-HCL at a final concentration of 20mM, NaCL at a final concentration of 0.2M and imidazole at a final concentration of 10mM were added, fully stirred, then centrifuged again at 12000g of centrifugal force, and the supernatant was collected and used as a crude extract containing the recombinant CTB fusion protein (rCTB4573₃-OPS_{*Spty*50973}) that was modified by *Salmonella paratyphi A* O-polysaccharide.

### 2) Purifying samples with Chelating affinity chromatographic column

Chelating affinity chromatographic column (Φ 1.6cm^{∗}15cm) was used for primary purification of samples. The column bed was first rinsed with 3 column bed volumes of 0.5M NaOH, then equilibrated with deionized water to neutral pH, then equilibrated with 3 column bed volumes of 0.5M NiSO₄, and then equilibrated with 1 column bed volume of B1 solution (20mM pH 7.5 Tris-HCl, 0.5M NaCl, 500mM imidazole), and finally equilibrated with 3 column bed volumes of A1 solution (20mM pH 7.5 Tris-HCl, 0.5M NaCl, 10mM imidazole), wherein the above-used flow rate was always 4 mL/min.

The above crude extract containingrCTB4573₃-OPS_{*Spty*50973} was loaded from A tube, and the unbound protein was washed off with solution A, and the elution was finally performed by using 100% B1 to collect 30 mL of eluate.

### 3) Desalting samples

The sample that was preliminarily purified by the Chelating affinity chromatographic column was desalted by using G25 fine chromatographic column (Φ1.6cm ^{∗} 30cm), in which the mobile phase was A2 solution (20mM pH 5.4HAc-NaAc). The column bed was firstly rinsed with3 column bed volumes of 0.5M NaOH, then equilibrated with deionized water to pH neutral, and finally equilibrated with 3 column volumes of A2 solution. The sample was loaded from A tube, 60 mL of sample was collected, and the above-used flow rate was always 4 mL/min.

### 4) Further purification of rCTB4573₃-OPS_{Spty50973} by using ProteinPak SP8HR cation exchange chromatographic column

The desalted sample was further purified by ProteinPak SP8HRcation exchange chromatographic column (waters).The column bed was first rinsed with 3 column bed volumes of 0.5M NaOH, then equilibrated with deionized water to pH neutral, and then equilibrated with 3 column bed volumes of A2 solution (20mM pH5.4 HAc-NaAc). The sample was loaded from A tube, the unbound glycoprotein was washed off with A2 solution, then linear elution was performed using 0-50% B2 solution (20mM pH5.4 HAc-NaAc, 1M NaCl) for 30 min, and the eluate was collected, in which the above used flow rate was always 1 mL/min. The peak position of glycoprotein rCTB4573₃-OPS_{*Sp*50973} was at position of about 35-45mS/cm. The sample was analyzed by 12% SDS-PAGE and western blot, and the results were shown in Fig.6.

### VII. Preparation and animal experimental evaluation of polysaccharide-protein conjugate vaccines of rCTB4573₃-OPS_{Spty50973} and rEPA4573-OPS_{Spty50973}

### 1. Preparation of polysaccharide-protein conjugate vaccines of rCTB4573₃-OPS_{Spty50973} and rEPA4573-OPS_{Spty50973}

The purified rCTB4573₃-OPS_{*Spty*50973} and rEPA4573-OPS_{*Spty*50973} were sterilized by filtration, and mixed with aluminum hydroxide adjuvant (Rehydragel LV, General Chemical) at a ratio of 9:1.

### 2. Preparation of O-antigen (OPS_{Spty50973})

LPS was firstly extracted by hot phenol-water method (SUN Yang, FENG Shuzhang, ZHU Lingwei, et al, "Preparation and identification of Enterohemorrhagic Escherichia coli 0157 LPS monoclonal antibody, [J]. Journal of Zoonoses of China, 2007, 23 (10): 971-973), preserved by lyophilization, then dissolved with 1% glacial acetic acid at a concentration of 10 mg/ml, treated with boiling water bath for 90 minutes, then cooled to room temperature, and adjusted to pH 7.0. The supernatant was collected after centrifugation at 64,000×gfor 5 hours, and thoroughly dialyzed with deionized water and preserved by lyophilization.

### 3. Animal immunization and effect evaluation of conjugate vaccines of rCTB4573₃-OPS_{Spty50973} and rEPA4573-OPS_{Spty50973}

40 female Balb/c mice of 6 weeks old were randomly divided into 4 groups. Aluminum hydroxide, OPS_{*Spty*50973},rCTB4573₃-OPS_{*Spty*50973} and rEPA4573-OPS_{*Spty*50973} samples were separately injected into muscles of the 4 group of mice, in which the aluminum hydroxide group was negative control, the other three groups were injected with 10µg polysaccharide in an amount expressed in polysaccharide content; and blood samples were taken separately on the 1^{st}, 22^{nd} and 50^{th} day after immunization, and on the 10^{th} day after the third immunization.

The antibody titer of anti-*Salmonella paratyphi A* O-polysaccharide in mice serum of each group was measured by indirect ELISA method. The enzyme-linked plate was coated with the extracted *Salmonella paratyphi A* LPS, in which each well was coated with 10µg of LPS, and other procedures could be seen in "A Guide to Experimental Biomedical Biology".

It can be seen from Fig.7, both of therCTB4573₃-OPS_{*Spty*50973} and rEPA4573-OPS_{*Spty*50973} as prepared in the present invention via extending OPS of *Salmonella paratyphi A* CMCC50973can induce mice to produce specific antibodies against *Salmonella paratyphi A* CMCC50973 OPS, in which the antibody titer increased significantly in comparison with the OPS group with injection of OPS only.

### Industrial Applications

In comparison with Paratyphoid A polysaccharide-protein conjugate vaccines in the prior art, the present invention prepares Paratyphoid A polysaccharide-protein conjugate vaccines by using an O-antigen with extended carbohydrate chain by one-step bio-crosslinking method. The method comprises: using *Salmonella paratyphi A* as a host strain wherein the O-antigen chain length was extended and O-antigen ligase gene *waaL* was deleted, co-expressing a recombinant fusion protein gene and a *Neisseria meningitidis* O-oligosaccharide transferase gene *pglL* in the host strain, using the O-antigen of the host strain *Salmonella paratyphi A* to directly modify the recombinant fusion protein in manner of O-glycosylation modification, so as to obtain O-antigen-modified recombinant fusion protein of *Salmonella paratyphi A* and to prepare Paratyphoid A polysaccharide-protein conjugate vaccines rCTB4573₃-OPS_{*Spty*50973} and rEPA4573-OPS_{*Spty*50973}. The results showed that the vaccines prepared by the present invention can induce in mice the generation of specific antibody against *Salmonella paratyphi A* CMCC50973 OPS, and the antibody titer is obviously improved in comparison with the mice with injection of OPS alone.

Unless otherwise indicated, all technical and scientific terms used herein have the same meaning as commonly understood by one of ordinary skill in the art to which this invention pertains. Exemplary methods and materials are described below. Although methods and materials similar or equivalent to those described in the text can also be used to carry out the present invention, they are obvious for those skilled in the art.. In the event of inconsistencies, the present specification including definitions should prevail. All materials, methods and examples above mentioned are illustrative only and not restrictive.

## Claims

1. A recombinant strain, which is obtained by introducing a *cld*_{LT2} gene encoding an enzyme controlling chain length of O-antigen of *Salmonella typhimurium* into *Salmonella paratyphi A* which is deficient in *cld* gene encoding an enzyme controlling chain length of O-antigen;
wherein said *cld*_{LT2} gene encodes the enzyme controlling chain length of O-antigen of *Salmonella typhimurium* which has an amino acid sequence as shown in SEQ ID NO: 2;
wherein said *cld* gene has a sequence as shown by sites from 887812 to 888789 of the sequence with accession number CP000026 in GenBank.

2. The recombinant strain according to claim 1, **characterized in that** the gene encoding the enzyme controlling chain length of O-antigen of *Salmonella typhimurium* has a sequence as shown in SEQ ID NO: 1.

3. The recombinant strain according to claim 1, **characterized in that** the *Salmonella paratyphi A* which is deficient in *cld* gene encoding an enzyme controlling chain length of O-antigen is obtained by a method comprising the following steps:
(1) preparing a linear targeting DNA fragment 1, which has a nucleotide sequence as shown in SEQ ID NO: 11, and contains a *cat* gene;
(2) transforming pKD46 plasmid into a *Salmonella paratyphi* strain, to obtain a recombinant strain designated as *S. paratyphi*/pKD46;
(3) inducing expression of Red recombination system in the *S. paratyphi*/pKD46 strain, and transforming the linear targeting DNA fragment 1 into the *S*. *paratyphi*/pKD46 strain, so that the linear targeting DNA fragment 1 replaces the *cld* gene of the *S. paratyphi*/pKD46 strain to obtain a recombinant strain designated as *S. paratyphicld::cat*/pKD46;
(4) deleting the pKD46 plasmid from the *S. paratyphi cld::cat*/pKD46 strain, to obtain a recombinant strain designated as *S. paratyphi cld::cat*;
the *S. paratyphi cld::cat* is a *S. paratyphi* in which *cld* gene sequence is substituted with *cat* gene sequence;
(5) transforming plasmid pCP20 into the *S. paratyphi cld::cat* and deleting *cat* gene, to obtain a recombinant strain designated as *S. paratyphi Δcld;*
the *S. paratyphi Δcld* is a *cld* gene-deleted *S. paratyphi.*

4. A method for extending carbohydrate chain length of O-antigen of *Salmonella paratyphi A,* comprising the following steps: culturing the recombinant strain according to claim 1 to express the *cld*_{LT2} gene, so that the recombinant strain synthesizes an O-antigen of which carbohydrate chain length is extended.

5. A method for preparing a vaccine for prevention and/or treatment of a disease caused by *Salmonella paratyphi A* by one-step bio-crosslinking, comprising the steps of:
(1) inactivating *cld* gene encoding an enzyme controlling chain length of O-antigen and *waaL* gene encoding O-antigen ligase of a *Salmonella paratyphi A* strain, to obtain a *Salmonella paratyphi A* with double deletion of *cld* gene and *waaL* gene; wherein said *cld* gene has a sequence as shown by sites from 887812 to 888789 of the sequence with accession number CP000026 in GenBank;
(2) introducing *cld*_{LT2} gene encoding an enzyme controlling chain length of O-antigen of *Salmonella typhimurium*, *pglL* gene encoding O-oligosaccharyltransferase of *Neisseria meningitidis* and a gene coding recombinant fusion protein into the *Salmonella paratyphi A* with double deletion of *cld* gene and *waaL* gene, to obtain a recombinant strain; wherein said *cld*_{LT2} gene encodes the enzyme controlling chain length of O-antigen of *Salmonella typhimurium* which has an amino acid sequence as shown in SEQ ID NO: 2;
(3) culturing the recombinant strain to obtain an O-antigen-modified recombinant fusion protein and processing the O-antigen-modified recombinant fusion protein to obtain the desired vaccine;
wherein the recombinant fusion protein comprises a N-terminal signal peptide, a carrier protein, and a peptide fragment comprising a serine as an O-glycosylation site at position 63 of *Neisseria meningitidis* pilin PilE;
the N-terminal signal peptide is PelB signal peptide, DsbA signal peptide, STII signal peptide, OmpA signal peptide, PhoA signal peptide, LamB signal peptide, SpA signal peptide or Enx signal peptide, preferably a DsbA signal peptide;
the carrier protein is a non-toxic mutant of a bacterial toxin protein or a fragment of a bacterial toxin protein;
the peptide fragment comprising a serine as an O-glycosylation site at position 63 of *Neisseria meningitidis* pilin PilE is a peptide fragment with an amino acids as set forth in positions 45-73 of *Neisseria meningitidis* pilin PilE.

6. The method according to claim 5, **characterized in that**, the *Salmonella paratyphi A* with double deletion of *cld* gene and *waaL* gene is constructed by a method comprising the following steps:
(1) preparing a linear targeting DNA fragment 2, which has a nucleotide sequence as shown in SEQ ID NO: 12, and which contains a *kan* gene;
(2) transforming pKOBEG plasmid into the *Salmonella.Paratyphi*Δ*cld* strain as defined in claim 3, to obtain a recombinant strain designated as *S. paratyphi*Δ*cld*/pKOBEG;
(3) inducing expression of Red recombination system in the *S. paratyphi*Δ*cld*/pKOBEG strain, and transforming the linear targeting DNA fragment 2 into the *S. paratyphi*Δ*cld*/pKOBEG strain, so that the linear targeting DNA fragment 2 replaces the *waaL* gene of the *S. paratyphi*Δ*cld*/pKOBEG strain to obtain a recombinant strain designated as *S. paratyphi*Δ*cld waal::kan*/pKOBEG;
(4) deleting the pKOBEG plasmid from the *S. paratyphi*Δ*cld waal::kan*/pKOBEG strain, to obtain a recombinant strain designated as *S. paratyphi*Δ*cldwaaL::kan*;
the *S. paratyphi*Δ*cldwaaL::kan* is a *S*.*paratyphi*Δ*cld* in which *waaL* gene sequence is substituted with *kan* gene sequence;
(5) transforming plasmid pCP20 into the *S*. *paratyphi*Δ*cldwaaL::kan* and deleting *kan* gene, to obtain a recombinant strain designated as *S*. *paratyphi*Δ*cld*Δ*waaL*;
the *S*. *paratyphi*Δ*cld*Δ*waaL* is a *S. paratyphi* with deletion of *cld* gene and *waaL* gene.

7. The method according to claim 5, **characterized in that**, the bacterial toxin protein is *Pseudomonas aeruginosa* exotoxin A, cholera toxin, diphtheria toxin or tetanus toxin.

8. The method according to claim 5, **characterized in that**, the non-toxic mutant of the bacterial toxin protein is a non-toxic mutant of *Pseudomonas aeruginosa* exotoxin A or a non-toxic mutant of diphtheria toxin;
the fragment of the bacterial toxin protein is a B subunit of cholera toxin or a C protein of tetanus toxin.

9. The method according to claim 5, **characterized in that**,
the *cld*_{LT2} gene encoding an enzyme controlling chain length of O-antigen of *Salmonella typhimurium* and the *pglL* gene encoding O-oligosaccharide transferase of *Neisseria meningitidis* are introduced into the *Salmonella paratyphi A* with double deletion of *cld* gene and *waaL* gene through pETtac28*-pglL-cld*_{LT2} recombinant expression vector;
the gene coding the recombinant fusion protein is introduced into the *Salmonella paratyphi A* with double deletion of *cld* gene and *waaL* gene through a pMMB66EH-rCTB4573 recombinant expression vector or a pMMB66EH-rEPA4573 recombinant expression vector or a pMMB66EH-rCTB4573₃ recombinant expression vector;
the pETtac28*-pglL-cld*_{LT2} recombinant expression vector is constructed by a method comprising: the *cld*_{LT2} gene encoding an enzyme controlling chain length of O-antigen of *Salmonella typhimurium* and the pMMB66EH vector are digested by restriction endonucleases EcoR I and Hind III, and are ligated to obtain a transition vector pMMB66EH-*cld*_{LT2}; transition vector pMMB66EH-*cld*_{LT2} is used as template, and an expression cassette of *cld*_{LT2} is obtained by amplification; the expression cassette of *cld*_{LT2} and pET28a vector are digested with restriction endonuclease Xba I and Xho I, and are ligated to obtain pETtac28-*cld*_{LT2} vector; *pglL* gene encoding O-oligosaccharide transferase of *Neisseria meningitidis* and pKK223-3 vector are digested by restriction endonucleases EcoR I and Hind III, and are ligated to obtain pKK223-3-*pglL* vector; pKK223-3-*pglL* vector is used as template, and an expression cassette of *PglL* is obtained by amplification; the expression cassette of PglL and the pETtac28-*cld*_{LT2} vector are digested by restriction endonuclease Bgl II, and are ligated to obtain the pETtac28-*pglL*-*cld*_{LT2} recombinant expression vector;
the pMMB66EH-rEPA4573 recombinant expression vector is constructed by a method comprising: ligating the gene encoding fusion protein rEPA4573 of recombinant *Pseudomonas aeruginosa* exotoxin A into a multiple cloning site of pMMB66EH vector to obtain the pMMB66EH-rEPA4573 recombinant expression vector;
the pMMB66EH-rCTB4573 recombinant expression vector is constructed by a method comprising: the gene encoding fusion protein rCTB4573 of recombinant cholera toxin B subunit is ligated into a multiple cloning site of pMMB66EH vector to obtain the pMMB66EH-rCTB4573 recombinant expression vector;
the pMMB66EH-rCTB4573₃ recombinant expression vector is constructed by a method comprising: the gene encoding fusion protein rCTB4573₃ of recombinant cholera toxin B subunit is ligated into a multiple cloning site of pMMB66EH vector to obtain the pMMB66EH-rCTB4573₃ recombinant expression vector;
wherein:
the enzyme controlling chain length of O-antigen of *Salmonella typhimurium* has an amino acid sequence as shown in SEQ ID NO: 2;
the *Neisseria meningitidis* oligosaccharyltransferase pglL has an amino acid sequence as shown in SEQ ID NO: 8;
the fusion protein rEPA4573 of recombinant *Pseudomonas aeruginosa* exotoxin A has an amino acid sequence as shown in SEQ ID NO: 4;
the fusion protein rCTB4573 of recombinant cholera toxin B subunit has an amino acid sequence as shown in SEQ ID NO: 6;
the fusion protein rCTB4573₃ of recombinant cholera toxin B subunit has an amino acid sequence as shown in SEQ ID NO: 10;
the peptide with amino acid sequence as set forth in position 45-73 of *Neisseria meningitidis* pilin PilE has an amino acid sequence as shown in SEQ ID NO: 14.

## Patentansprüche

1. Rekombinanter Stamm, welcher erhalten wird durch Einführung eines *cld*_{LT2}*-Gens*, das für ein Enzym kodiert, welches die Kettenlänge des O-Antigens von *Salmonella typhimurium* kontrolliert, in *Salmonella paratyphi A*, dem das *cld*-Gen, das für ein Enzym kodiert, welches die Kettenlänge des O-Antigens kontrolliert, fehlt;
wobei das *cld*_{LT2}-Gen für das Enzym kodiert, welches die Kettenlänge des O-Antigens von *Salmonella typhimurium* kontrolliert, das eine Aminosäuresequenz aufweist, wie in SEQ ID NO: 2 dargestellt;
wobei das *cld*-Gen eine Sequenz aufweist, wie an den Stellen 887812 bis 888789 der Sequenz mit der Zugangsnummer CP000026 in der GenBank gezeigt.

2. Rekombinanter Stamm nach Anspruch 1, **dadurch gekennzeichnet, dass** das Gen, das für das Enzym kodiert, welches die Kettenlänge des O-Antigens von *Salmonella typhimurium* kontrolliert, eine Sequenz aufweist, wie in SEQ ID NO: 1 dargestellt.

3. Rekombinanter Stamm nach Anspruch 1, **dadurch gekennzeichnet, dass** das *Salmonella paratyphi A*, welchem das *cld*-Gen, das für ein Enzym kodiert, welches die Kettenlänge des O-Antigens kontrolliert, fehlt, durch ein Verfahren erhalten wird, das die folgenden Schritte umfasst:
(1) Herstellung eines linearen Targeting-DNA-Fragments 1, das eine Nukleotidsequenz aufweist, wie in SEQ ID NO: 11 gezeigt, und ein *Katzen-Gen* enthält;
(2) Umwandlung des pKD46-Plasmids in einen *Salmonella paratyphi-*Stamm, um einen rekombinanten Stamm mit der Bezeichnung *S*. *paratyphi*/pKD46 zu erhalten;
(3) Induktion der Expression des Red-Rekombinationssystems im *S. paratyphi*/pKD46-Stamm und Transformation des linearen Targeting-DNA-Fragments 1 in den *S. paratyphi*/pKD46-Stamm, so dass das lineare Targeting-DNA-Fragment 1 das *cld*-Gen des *S. paratyphi*/pKD46-Stammes ersetzt, um einen rekombinanten Stamm mit der Bezeichnung *S*. *paratyphi cld::cat*/*pKD46* zu erhalten;
(4) Deletion des pKD46-Plasmids aus dem *S. paratyphi cld::cat*/pKD46-Stamm, um einen rekombinanten Stamm mit der Bezeichnung *S*. *paratyphi cld::cat* zu erhalten;
der *S*. *paratyphi cld::cat* ist ein *S. paratyphi,* bei dem die *cld*-Gensequenz durch *Katzen-*Gensequenz ersetzt ist;
(5) Transformation des Plasmids pCP20 in *S. paratyphi cld::cat* und Deletion des *Katzen-*Gens, um einen rekombinanten Stamm mit der Bezeichnung *S*. *paratyphiΔcld* zu erhalten;
der *S*. *paratyphiΔcld* ist ein *S*. *paratyphi,* mit entferntem *cld*-Gen.

4. Verfahren zur Verlängerung der Kohlenhydratkettenlänge des O-Antigens von *Salmonella paratyphi A*, umfassend die folgenden Schritte: Kultivierung des rekombinanten Stammes nach Anspruch 1 zur Expression des *cld*_{LT2}-Gens, so dass der rekombinante Stamm ein O-Antigen synthetisiert, dessen Kohlenhydratkettenlänge verlängert wird.

5. Verfahren zur Herstellung eines Impfstoffs zur Prävention und/oder Behandlung einer durch *Salmonella paratyphi A* verursachten Erkrankung durch einstufige Bio-Vernetzung, umfassend die folgenden Schritte:
(1) Inaktivierung des *cld*-Gens, das für ein Enzym kodiert, welches die Kettenlänge des O-Antigens kontrolliert, und des *waaL-Gens,* das für die O-Antigen-Ligase eines *Salmonella paratyphi* A-Stammes kodiert, um einen *Salmonella paratyphi A* mit Doppel-Deletion des *cld-*Gens und des *waaL-Gens* zu erhalten; wobei das *cld*-Gen eine Sequenz aufweist, wie an den Stellen 887812 bis 888789 der Sequenz mit der Zugangsnummer CP000026 in der GenBank gezeigt;
(2) Einführung des *cld*_{LT2}-Gens, das für ein Enzym kodiert, welches die Kettenlänge des O-Antigens von *Salmonella typhimurium* kontrolliert, des *pglL*-Gens, das für die O-Oligosaccharyltransferase von *Neisseria meningitidis* kodiert, und eines Gens, das für ein rekombinantes Fusionsprotein kodiert, in das *Salmonella paratyphi A* mit Doppel-Deletion des *cld*-Gens und des *waaL-Gens,* um einen rekombinanten Stamm zu erhalten; wobei das *cld*_{LT2}-Gen für das Enzym kodiert, welches die Kettenlänge des O-Antigens von *Salmonella typhimurium* kontrolliert, das eine Aminosäuresequenz wie in SEQ ID NO: 2 gezeigt, aufweist;
(3) Kultivierung des rekombinanten Stammes, um ein O-Antigen-modifiziertes rekombinantes Fusionsprotein zu erhalten, und Verarbeitung des O-Antigen-modifizierten rekombinanten Fusionsproteins, um den gewünschten Impfstoff zu erhalten;
wobei das rekombinante Fusionsprotein ein N-terminales Signalpeptid, ein Trägerprotein und ein Peptidfragment umfasst, das ein Serin als O-Glykosylierungsstelle an Position 63 von *Neisseria meningitidis* Pilin PilE umfasst;
wobei das N-terminale Signalpeptid ein PelB-Signalpeptid, DsbA-Signalpeptid, STII-Signalpeptid, OmpA-Signalpeptid, PhoA-Signalpeptid, LamB-Signalpeptid, SpA-Signalpeptid oder Enx-Signalpeptid, vorzugsweise ein DsbA-Signalpeptid, ist;
wobei das Trägerprotein eine nicht toxische Mutante eines bakteriellen Toxinproteins oder ein Fragment eines bakteriellen Toxinproteins ist;
wobei das Peptidfragment, das ein Serin als O-Glykosylierungsstelle an Position 63 von *Neisseria meningitidis* Pilin PilE umfasst, ein Peptidfragment mit einer Aminosäure ist, wie sie in den Positionen 45-73 von *Neisseria meningitidis* pilin PilE gegeben ist.

6. Verfahren nach Anspruch 5, **dadurch gekennzeichnet, dass** das *Salmonella paratyphi A* mit Doppel-Deletion des *cld-Gens* und des *waaL-Gens* nach einem Verfahren konstruiert wird, das die folgenden Schritte umfasst:
(1) Herstellung eines linearen Targeting-DNA-Fragments 2, das eine Nukleotidsequenz wie in SEQ ID NO: 12 gezeigt, aufweist, und das ein *kan*-Gen enthält;
(2) Umwandlung von pKOBEG-Plasmid in den *Salmonella.ParatyphiΔcld-*Stamm gemäß Anspruch 3, um einen rekombinanten Stamm mit der Bezeichnung *S. paratyphiΔcld*/pKOBEG zu erhalten;
(3) Induktion der Expression des Red-Rekombinationssystems in dem *S. paratyphiΔcld*/pKOBEG*-*Stamm und Transformation des linearen Targeting-DNA-Fragments 2 in den *S. paratyphiΔcld*/pKOBEG*-*Stamm, so dass das linearen Targeting-DNA-Fragment 2 das *waaL*-Gen des *S. paratyphiΔcld*/pKOBEG-Stamms ersetzt, um einen rekombinanten Stamm mit der Bezeichnung *S. paratyphiΔcld waal::kan*/pKOBEG zu erhalten;
(4) Deletion des pKOBEG-Plasmids aus dem S. *paratyphiΔcld waal::kan*/pKOBEG-Stamm, um einen rekombinanten Stamm mit der Bezeichnung *S*. *paratyphiΔcldwaaL::kan* zu erhalten; *S. paratyphiΔcldwaaL::kan* ist ein *S*.*paratyphiΔcld*, bei welchem die *waaL*-Gensequenz durch die *kan*-Gensequenz ersetzt ist;
(5) Transformation des Plasmids pCP20 in *S*. *paratyphiΔcldwaaL::kan* und Deletion des *kan-*Gens, um einen rekombinanten Stamm mit der Bezeichnung *S*. *paratyphiΔcldΔwaaL* zu erhalten;
*S*. *paratyphiΔcldΔwaaL* ist ein *S*. *paratyphi* mit Deletion des *cld*-Gens und des *waaL-Gens.*

7. Verfahren nach Anspruch 5, **dadurch gekennzeichnet, dass** das bakterielle Toxinprotein ein *Pseudomonas aeruginosa* Exotoxin A, Cholera-Toxin, Diphtherie-Toxin oder Tetanus-Toxin ist.

8. Verfahren nach Anspruch 5, **dadurch gekennzeichnet, dass** die nicht toxische Mutante des bakteriellen Toxinproteins eine nicht toxische Mutante von *Pseudomonas aeruginosa* Exotoxin A oder eine nicht toxische Mutante von Diphtherietoxin ist;
und dass das Fragment des bakteriellen Toxinproteins eine B-Untereinheit des Cholera-Toxins oder ein C-Protein des Tetanus-Toxins ist.

9. Verfahren nach Anspruch 5, **dadurch gekennzeichnet, dass**,
das *cld*_{LT2}-Gen, das für ein Enzym kodiert, welches die Kettenlänge des O-Antigens von *Salmonella typhimurium* kontrolliert, und das *pglL-*Gen, welches für die O-OligosaccharidTransferase von *Neisseria meningitidis* kodiert, durch den rekombinanten Expressionsvektor pETtac28-*pglL*-*cld*_{LT2} in den *Salmonella paratyphi A* mit Doppel-Deletion des *cld*-Gens und des *waaL-Gens* eingeführt wird;
das Gen, welches für das rekombinante Fusionsprotein kodiert, durch einen rekombinanten Expressionsvektor pMMB66EH-rCTB4573 oder einen rekombinanten Expressionsvektor pMMB66EH-rEPA4573 oder einen rekombinanten Expressionsvektor pMMB66EH-rCTB4573₃ in das *Salmonella paratyphi A* mit Doppel-Deletion des *cld-Gens* und des *waaL-Gens* eingeführt wird;
der rekombinante Expressionsvektor *pETtac28-pglL-cld*_{LT2} nach einem Verfahren konstruiert wird, welches Folgendes umfasst: das *cld*_{LT2}-Gen, das für ein Enzym kodiert, welches die Kettenlänge des O-Antigens von *Salmonella typhimurium* kontrolliert, und der pMMB66EH-Vektor werden von den Restriktionsendonukleasen EcoR I und Hind III verdaut und ligiert, um einen Übergangsvektor pMMB66EH-*cld*_{LT2} zu erhalten; der Übergangsvektor pMMB66EH-*cld*_{LT2} wird als Template verwendet, und eine Expressionskassette von *cld*_{LT2} wird durch Amplifikation erhalten; die Expressionskassette von *cld*_{LT2} und der pET28a-Vektor werden mit der Restriktionsendonuklease Xba I und Xho I verdaut und ligiert, um den pETtac28-*cld*_{LT2}-Vektor zu erhalten; das *pglL*-Gen, das für die O-Oligosaccharidtransferase von *Neisseria meningitidis* kodiert, und der pKK223-3-Vektor werden von den Restriktionsendonukleasen EcoR I und Hind III verdaut und ligiert, um den pKK223-3-*pglL-*Vektor zu erhalten; der pKK223-3-*pglL*-Vektor wird als Template verwendet, und eine Expressionskassette von *PglL* wird durch Amplifikation erhalten; die Expressionskassette von PglL und der pETtac28-*cld*_{LT2} Vektor werden durch die Restriktionsendonuklease Bgl II verdaut und ligiert, um den rekombinanten pETtac28-*pglL*-*cld*_{LT2} Expressionsvektor zu erhalten;
der rekombinante Expressionsvektor pMMB66EH-rEPA4573 durch ein Verfahren konstruiert wird, welches Folgendes umfasst: Ligieren des Gens, das für das Fusionsprotein rEPA4573 des rekombinanten *Pseudomonas aeruginosa*-Exotoxins A kodiert, in eine multiple Klonierungsstelle des pMMB66EH-Vektors, um den rekombinanten Expressionsvektor pMMB66EH-rEPA4573 zu erhalten;
der rekombinante Expressionsvektor pMMB66EH-rCTB4573 nach einem Verfahren konstruiert wird, das Folgendes umfasst: das Gen, das für das Fusionsprotein rCTB4573 der rekombinanten Choleratoxin-B-Untereinheit kodiert, wird in eine multiple Klonierungsstelle des pMMB66EH-Vektors ligiert, um den rekombinanten Expressionsvektor pMMB66EH-rCTB4573 zu erhalten;
der rekombinante Expressionsvektor pMMB66EH-rCTB4573₃ wird nach einem Verfahren konstruiert, das Folgendes umfasst: das für das Fusionsprotein rCTB4573₃ der rekombinanten Choleratoxin-B-Untereinheit kodierende Gen wird in eine multiple Klonierungsstelle des pMMB66EH-Vektors ligiert, um den rekombinanten Expressionsvektor pMMB66EH-rCTB4573₃ zu erhalten;
wobei:
das Enzym, das die Kettenlänge des O-Antigens von *Salmonella typhimurium* kontrolliert, eine Aminosäuresequenz, wie in SEQ ID NO: 2 gezeigt, aufweist;
die *Neisseria* meningitidis-Oligosaccharyltransferase pglL eine Aminosäuresequenz, wie in SEQ ID NO: 8 gezeigt, aufweist;
das Fusionsprotein rEPA4573 des rekombinanten *Pseudomonas* aeruginosa-Exotoxins A eine Aminosäuresequenz, wie in SEQ ID NO: 4 gezeigt, aufweist;
das Fusionsprotein rCTB4573 der rekombinanten Choleratoxin-B-Untereinheit eine Aminosäuresequenz, wie in SEQ ID NO: 6 gezeigt, aufweist;
das Fusionsprotein rCTB4573₃ der rekombinanten Choleratoxin-B-Untereinheit eine Aminosäuresequenz, wie in SEQ ID NO: 10 gezeigt, aufweist;
das Peptid mit der Aminosäuresequenz, wie an der Position 45-73 von *Neisseria meningitidis* Pilin PilE dargestellt, eine Aminosäuresequenz, wie in SEQ ID NO: 14 gezeigt, aufweist.

## Revendications

1. Souche recombinante, qui est obtenue en introduisant un gène *cld*_{LT2} codant pour une enzyme contrôlant la longueur de la chaîne de l'antigène O de *Salmonella typhimurium* dans *Salmonella paratyphi A* qui est déficiente en gène *cld* codant pour une enzyme contrôlant la longueur de la chaîne de l'antigène O ;
dans laquelle ledit gène *cld*_{LT2} code l'enzyme contrôlant la longueur de la chaîne de l'antigène O de *Salmonella typhimurium* qui a une séquence d'acides aminés telle que montrée dans la SEQ ID NO : 2 ;
dans laquelle ledit gène *cld* a une séquence telle que montrée par les sites de 887812 à 888789 de la séquence ayant le numéro d'accès CP000026 dans GenBank.

2. Souche recombinante selon la revendication 1, **caractérisée en ce que** le gène codant pour l'enzyme contrôlant la longueur de la chaîne de l'antigène O de *Salmonella typhimurium* a une séquence telle que montrée dans la SEQ ID NO : 1.

3. Souche recombinante selon la revendication 1, **caractérisée en ce que** la *Salmonella paratyphi A* qui est déficiente en gène *cld* codant pour une enzyme contrôlant la longueur de la chaîne de l'antigène O est obtenue par une méthode comprenant les étapes suivantes :
(1) préparer un fragment d'ADN de ciblage linéaire 1, qui a une séquence de nucléotides telle que montrée dans SEQ ID NO : 11, et contient un gène de *chat;*
(2) transformer du plasmide pKD46 en une souche de *Salmonella paratyphi,* pour obtenir une souche recombinante désignée par *S*. *paratyphi*/pKD46 ;
(3) induire l'expression du système de recombinaison Red dans la souche *S. paratyphi*/pKD46, et transformer le fragment d'ADN de ciblage linéaire 1 en la souche S. *paratyphi*/pKD46, de sorte que le fragment d'ADN de ciblage linéaire 1 remplace le gène *cld* de la souche *S*. *paratyphi*/pKD46 pour obtenir une souche recombinante désignée par *S. paratyphi cld::cat*/*pKD46* ;
(4) supprimer le plasmide pKD46 de la souche *S*. *paratyphi cld::cat*/pKD46, pour obtenir une souche recombinante désignée par *S*. *paratyphi cld::cat* ;
le *S*. *paratyphi cld::cat* est un *S*. *paratyphi* dans lequel la séquence du gène *cld est* substituée par la séquence du gène du *chat;*
(5) transformer le plasmide pCP20 en *S*. *paratyphi cld::cat* et supprimer le gène du *chat*, pour obtenir une souche recombinante désignée par *S*. *paratyphi Δcld* ;
le *S*. *paratyphi Δcld* est un *S*. *paratyphi dont le* gène *cld a été* supprimé.

4. Procédé pour allonger la longueur de la chaîne hydrocarbonnée de l'antigène O de *Salmonella paratyphi A*, comprenant les étapes suivantes : cultiver la souche recombinante selon la revendication 1 pour exprimer le gène *cld*_{LT2}, de sorte que la souche recombinante synthétise un antigène O dont la longueur de la chaîne hydrocarbonnée est allongée.

5. Méthode de préparation d'un vaccin pour la prévention et/ou le traitement d'une maladie causée par *Salmonella paratyphi A* par une bio-réticulation en une étape, comprenant les étapes suivantes
(1) inactiver le gène *cld* codant pour une enzyme contrôlant la longueur de la chaîne de l'antigène O et du gène *waaL* codant pour la ligase de l'antigène O d'une souche de *Salmonella paratyphi A*, pour obtenir une *Salmonella paratyphi A* avec une double délétion du gène *cld* et du gène *waaL* ; dans laquelle ledit gène *cld* a une séquence telle que montrée par les sites de 887812 à 888789 de la séquence ayant le numéro d'accès CP000026 dans GenBank;
(2) introduire le gène *cld*_{LT2} codant pour une enzyme contrôlant la longueur de la chaîne de l'antigène O de *Salmonella typhimurium,* le gène *pglL* codant pour la O-oligosaccharyltransférase de *Neisseria meningitidis* et un gène codant pour une protéine de fusion recombinante dans la *Salmonella paratyphiA* avec double délétion du gène *cld* et du gène *waaL,* pour obtenir une souche recombinante ; dans laquelle ledit gène *cld*_{LT2} code pour l'enzyme contrôlant la longueur de la chaîne de l'antigène O de *Salmonella typhimurium* qui a une séquence d'acides aminés telle que montrée dans la SEQ ID NO : 2 ;
(3) cultiver la souche recombinante pour obtenir une protéine de fusion recombinante modifiée par l'antigène O et traiter la protéine de fusion recombinante modifiée par l'antigène O pour obtenir le vaccin souhaité ;
dans laquelle la protéine de fusion recombinante comprend un peptide signal N-terminal, une protéine porteuse et un fragment peptidique comprenant une sérine comme site de O-glycosylation en position 63 de la piline PilE de *Neisseria* meningitidis ;
le peptide signal N-terminal est le peptide signal PelB, le peptide signal DsbA, le peptide signal STII, le peptide signal OmpA, le peptide signal PhoA, le peptide signal LamB, le peptide signal SpA ou le peptide signal Enx, de préférence un peptide signal DsbA ;
la protéine porteuse est un mutant non toxique d'une protéine de toxine bactérienne ou un fragment d'une protéine de toxine bactérienne ;
le fragment peptidique comprenant une sérine comme site de O-glycosylation à la position 63 de *Neisseria* meningitidis pilin PilE est un fragment peptidique avec un acide aminé tel que défini aux positions 45-73 de *Neisseria meningitidis* pilin PilE.

6. Méthode selon la revendication 5, **caractérisée en ce que**, la *Salmonella paratyphi A* avec double délétion du gène *cld* et du gène *waaL* est construite par une méthode comprenant les étapes suivantes :
(1) préparer un fragment d'ADN de ciblage linéaire 2, qui a une séquence de nucléotides telle que montrée dans SEQ ID NO : 12, et qui contient un gène *kan ;*
(2) transformer le plasmide pKOBEG en la souche *Salmonella.ParatyphiΔcld* telle que définie dans la revendication 3, pour obtenir une souche recombinante désignée par *S. ParatyphiΔcld*/pKOBEG ;
(3) en induisant l'expression du système de recombinaison Red dans la souche *S. paratyphiΔcld paratyphiΔcld*/pKOBEG, et en transformant le fragment d'ADN de ciblage linéaire 2 en souche S. *paratyphiΔcld*/pKOBEG, de sorte que le fragment d'ADN de ciblage linéaire 2 remplace le gène *waaL de* la souche *S*. *paratyphiΔcld*/pKOBEG pour obtenir une souche recombinante désignée par *S*. *paratyphiΔcld waal::kan*/pKOBEG ;
(4) supprimer le plasmide pKOBEG de la souche *S*. *paratyphiΔcld waal::kan*/pKOBEG, pour obtenir une souche recombinante désignée par *S*. *paratyphiΔcldwaaL::kan* ;
le *S*. *paratyphiΔcldwaaL::kan* est un *S.paratyphiΔcld* dans lequel la séquence du gène *waaL* est substituée par la séquence du gène *kan ;*
(5) transformer le plasmide pCP20 en *S*. *paratyphiΔcldwaaL::kan* et supprimer le gène *kan,* pour obtenir une souche recombinante appelée *S*. *paratyphiΔcldΔwaaL ;*
le *S*. *paratyphiΔcldΔwaaL* est un *S*. *paratyphi* avec délétion du gène *cld* et du gène *waaL.*

7. La méthode selon la revendication 5, **caractérisée en ce que** la protéine de la toxine bactérienne est l'exotoxine A de *Pseudomonas aeruginosa,* la toxine du choléra, la toxine de la diphtérie ou la toxine du tétanos.

8. Méthode selon la revendication 5, **caractérisée en ce que** le mutant non toxique de la protéine de la toxine bactérienne est un mutant non toxique de l'exotoxine A de *Pseudomonas aeruginosa* ou un mutant non toxique de la toxine diphtérique ;
le fragment de la protéine de la toxine bactérienne est une sous-unité B de la toxine du choléra ou une protéine C de la toxine du tétanos.

9. La méthode selon la revendication 5, **caractérisée en ce que**,
le gène *cld*_{LT2} codant pour une enzyme contrôlant la longueur de la chaîne de l'antigène O de *Salmonella typhimurium* et le gène *pglL* codant pour la O-oligosaccharide transférase de *Neisseria meningitidis* sont introduits dans la *Salmonella paratyphi A* avec une double délétion du gène *cld* et du gène *waaL* par le vecteur d'expression recombinant pETtac28-*pglL-cld*_{LT2} ;
le gène codant pour la protéine de fusion recombinante est introduit dans la *Salmonella paratyphi A* avec double délétion du gène *cld* et du gène *waaL* par un vecteur d'expression recombinant pMMB66EH-rCTB4573 ou un vecteur d'expression recombinant pMMB66EH-rEPA4573 ou un vecteur d'expression recombinant pMMB66EH-rCTB4573₃ ;
le vecteur d'expression recombinant pETtac28-*pglL*-*cld*_{LT2} est construit par une méthode comprenant : le gène *cld*_{LT2} codant pour une enzyme contrôlant la longueur de la chaîne de l'antigène O de *Salmonella typhimurium* et le vecteur pMMB66EH sont digérés par les endonucléases de restriction EcoR I et Hind III, et sont ligaturés pour obtenir un vecteur de transition pMMB66EH-*cld*_{LT2} ; le vecteur de transition pMMB66EH-*cld*_{LT2} est utilisé comme modèle, et une cassette d'expression de *cld*_{LT2} est obtenue par amplification ; la cassette d'expression de *cld*_{LT2} et le vecteur pET28a sont digérés par l'endonucléasesde restriction Xba I et Xho I, et sont ligaturés pour obtenir le vecteur pETtac28-*cld*_{LT2} ; le gène *pglL* codant pour la O-oligosaccharide transférase de *Neisseria meningitidis* et le vecteur pKK223-3 sont digérés par les endonucléases de restriction EcoR I et Hind III, et sont ligaturés pour obtenir le vecteur pKK223-3-*pglL* ; le vecteur pKK223-3-*pglL* est utilisé comme modèle, et une cassette d'expression de *PglL* est obtenue par amplification ; la cassette d'expression de *PglL* et le vecteur pETtac28-*cld*_{LT2} sont digérés par l'endonucléase de restriction Bgl II, et sont ligaturés pour obtenir le vecteur d'expression recombinant pETtac28-pglL-*cld*_{LT2} ;
le vecteur d'expression recombinant pMMB66EH-rEPA4573 est construit par une méthode comprenant : la ligature du gène codant pour la protéine de fusion rEPA4573 de l'exotoxine A recombinante de *Pseudomonas aeruginosa* dans un site de clonage multiple du vecteur pMMB66EH pour obtenir le vecteur d'expression recombinant pMMB66EH-rEPA4573 ;
le vecteur d'expression recombinant pMMB66EH-rCTB4573 est construit par une méthode comprenant : le gène codant pour la protéine de fusion rCTB4573 de la sous-unité B de la toxine du choléra recombinante est ligaturé dans un site de clonage multiple du vecteur pMMB66EH pour obtenir le vecteur d'expression recombinant pMMB66EH-rCTB4573 ;
le vecteur d'expression recombinant pMMB66EH-rCTB4573₃ est construit par une méthode comprenant : le gène codant pour la protéine de fusion rCTB45733 de la sous-unité B de la toxine du choléra recombinante est ligaturé dans un site de clonage multiple du vecteur pMMB66EH pour obtenir le vecteur d'expression recombinant pMMB66EH-rCTB4573₃ ;
où :
l'enzyme contrôlant la longueur de la chaîne de l'antigène O de *Salmonella typhimurium* a une séquence d'acides aminés telle que montré dans la SEQ ID NO : 2 ;
l'oligosaccharyltransferase pglL de *Neisseria meningitidis* a une séquence d'acides aminés telle que montrée dans la SEQ ID NO : 8 ;
la protéine de fusion rEPA4573 de l'exotoxine A recombinante de *Pseudomonas aeruginosa* a une séquence d'acides aminés telle que montrée dans la SEQ ID NO : 4 ;
la protéine de fusion rCTB4573 de la sous-unité B de la toxine recombinante du choléra a une séquence d'acides aminés telle que montrée dans la SEQ ID NO : 6 ;
la protéine de fusion rCTB45733 de la sous-unité B de la toxine recombinante du choléra a une séquence d'acides aminés telle que montrée dans la SEQ ID NO : 10 ;
le peptide avec la séquence d'acides aminés telle que présentée en position 45-73 de *Neisseria meningitidis* pilin PilE a une séquence d'acides aminés telle que montrée dans SEQ ID NO : 14.


### SEQUENCE LISTING

<110> Institute of Biotechnology Academy of Military Medical Sciences
   <120> A Salmonella Paratyphi A with an O-Antigen having an Extended
   Carbohydrate Chain and use thereof
<160> 14
<210> 1
   <211> 987bp
   <212> DNA
   <213> artificial
<220>
   <223>
<400> 1
   atgacagtgg acagtaatac cagcagtggc cgtggtaacg atcctgagca gatcgacctg      60
   attgagctgc tgctgcaact gtggcgtggc aagatgacca tcatcgtggc cgtgatcatc     120
   gccattctgc tggccgtggg ctatctgatg atcgccaagg aaaagtggac cagcaccgcc     180
   atcatcaccc agccggatgc cgcacaagtg gcaacctaca caaacgccct gaatgtgctg     240
   tacggcggta acgcaccgaa gattagtgag gtgcaggcca acttcatcag ccgtttcagc     300
   agcgcattca gcgccttaag cgaggtgctg gataaccaga aggagcgtga aaagctgacc     360
   atcgagcaga gcgtgaaagg tcaagccctg cctctgagcg ttagctatgt gagtaccacc     420
   gcagaaggtg cccaacgtcg tctggccgag tacatccaac aggtggatga agaggtggcc     480
   aaggaactgg aggtggatct gaaggacaac atcaccctgc agaccaagac cctgcaggag     540
   agcctggaaa cccaggaagt ggtggcccag gaacagaaag acctgcgcat caagcagatc     600
   gaagaggccc tgcgttacgc cgatgaagca aagatcaccc aaccgcagat ccaacagaca     660
   caagacgtga cccaggacac aatgttcctg ctgggtagcg acgccctgaa aagcatgatc     720
   cagaacgagg ccacacgtcc gctggtgttt agcccggcct actaccagac caaacagacc     780
   ctgctggata ttaagaacct gaaagtgacc gccgacaccg tgcacgtgta ccgctacgtg     840
   atgaaaccta ccctgccggt tcgtcgtgac agtcctaaga ccgcaatcac actggttctg     900
   gcagtgctgt taggcggtat gattggcgca ggcatcgtgt taggccgtaa cgccttacgc     960
   agctacaagc ctaaagcctt ataatga                                        987
<210> 2
   <211> 327
   <212> PRT
   <213> artificial
<220>
   <223>
<400> 2
   Met Thr Val Asp Ser Asn Thr Ser Ser Gly Arg Gly Asn Asp Pro Glu
   1               5                   10                  15
   Gln Ile Asp Leu Ile Glu Leu Leu Leu Gln Leu Trp Arg Gly Lys Met
               20                  25                  30
   Thr Ile Ile Val Ala Val Ile Ile Ala Ile Leu Leu Ala Val Gly Tyr
           35                  40                  45
   Leu Met Ile Ala Lys Glu Lys Trp Thr Ser Thr Ala Ile Ile Thr Gln
       50                  55                  60
   Pro Asp Ala Ala Gln Val Ala Thr Tyr Thr Asn Ala Leu Asn Val Leu
   65                  70                  75                  80
   Tyr Gly Gly Asn Ala Pro Lys Ile Ser Glu Val Gln Ala Asn Phe Ile
                   85                  90                  95
   Ser Arg Phe Ser Ser Ala Phe Ser Ala Leu Ser Glu Val Leu Asp Asn
               100                 105                 110
   Gln Lys Glu Arg Glu Lys Leu Thr Ile Glu Gln Ser Val Lys Gly Gln
           115                 120                 125
   Ala Leu Pro Leu Ser Val Ser Tyr Val Ser Thr Thr Ala Glu Gly Ala
       130                 135                 140
   Gln Arg Arg Leu Ala Glu Tyr Ile Gln Gln Val Asp Glu Glu Val Ala
   145                 150                 155                 160
   Lys Glu Leu Glu Val Asp Leu Lys Asp Asn Ile Thr Leu Gln Thr Lys
                   165                 170                 175
   Thr Leu Gln Glu Ser Leu Glu Thr Gln Glu Val Val Ala Gln Glu Gln
               180                 185                 190
   Lys Asp Leu Arg Ile Lys Gln Ile Glu Glu Ala Leu Arg Tyr Ala Asp
           195                 200                 205
   Glu Ala Lys Ile Thr Gln Pro Gln Ile Gln Gln Thr Gln Asp Val Thr
       210                 215                 220
   Gln Asp Thr Met Phe Leu Leu Gly Ser Asp Ala Leu Lys Ser Met Ile
   225                 230                 235                 240
   Gln Asn Glu Ala Thr Arg Pro Leu Val Phe Ser Pro Ala Tyr Tyr Gln
                   245                 250                 255
   Thr Lys Gln Thr Leu Leu Asp Ile Lys Asn Leu Lys Val Thr Ala Asp
               260                 265                 270
   Thr Val His Val Tyr Arg Tyr Val Met Lys Pro Thr Leu Pro Val Arg
           275                 280                 285
   Arg Asp Ser Pro Lys Thr Ala Ile Thr Leu Val Leu Ala Val Leu Leu
       290                 295                 300
   Gly Gly Met Ile Gly Ala Gly Ile Val Leu Gly Arg Asn Ala Leu Arg
   305                 310                 315                 320
   Ser Tyr Lys Pro Lys Ala Leu
                   325
<210> 3
   <211> 2025bp
   <212> DNA
   <213> artificial
<220>
   <223>
<400> 3
   atgaaaaaga tttggctggc gctggctggt ttagttttag cgtttagcgc atcggcggcc      60
   gaggaagcct tcgacctctg gaacgaatgc gccaaagcct gcgtgctcga cctcaaggac     120
   ggcgtgcgtt ccagccgcat gagcgtcgac ccggccatcg ccgacaccaa cggccagggc     180
   gtgctgcact actccatggt cctggagggc ggcaacgacg cgctcaagct ggccatcgac     240
   aacgccctca gcatcaccag cgacggcctg accatccgcc tcgaaggcgg cgtcgagccg     300
   aacaagccgg tgcgctacag ctacacgcgc caggcgcgcg gcagttggtc gctgaactgg     360
   ctggtaccga tcggccacga gaagccctcg aacatcaagg tgttcatcca cgaactgaac     420
   gccggtaacc agctcagcca catgtcgccg atctacacca tcgagatggg cgacgagttg     480
   ctggcgaagc tggcgcgcga tgccaccttc ttcgtcaggg cgcacgagag caacgagatg     540
   cagccgacgc tcgccatcag ccatgccggg gtcagcgtgg tcatggccca ggcccagccg     600
   cgccgggaaa agcgctggag cgaatgggcc agcggcaagg tgttgtgcct gctcgacccg     660
   ctggacgggg tctacaacta cctcgcccag cagcgctgca acctcgacga tacctgggaa     720
   ggcaagatct accgggtgct cgccggcaac ccggcgaagc atgacctgga catcaagccc     780
   acggtcatca gtcatcgcct gcacttcccc gagggcggca gcctggccgc gctgaccgcg     840
   caccaggctt gccacctgcc gctggagact ttcacccgtc atcgccagcc gcgcggctgg     900
   gaacaactgg agcagtgcgg ctatccggtg cagcggctgg tcgccctcta cctggcggcg     960
   cggctgtcgt ggaaccaggt cgaccaggtg atcggcaacg ccctggccag ccccggcagc    1020
   ggcggcgacc tgggcgaagc gatccgcgag cagccggagc aggcccgtct ggccctgacc    1080
   ctggccgccg ccgagagcga gcgcttcgtc cggcagggca ccggcaacga cgaggccggc    1140
   gcggccagcg ccgacgtggt gagcctgacc tgcccggtcg ccgccggtga atgcgcgggc    1200
   ccggcggaca gcggcgacgc cctgctggag cgcaactatc ccactggcgc ggagttcctc    1260
   ggcgacggcg gcgacatcag cttcagcacc cgcggcacgc agaactggac ggtggagcgg    1320
   ctgctccagg cgcaccgcca actggaggag cgcggctatg tgttcgtcgg ctaccacggc    1380
   accttcctcg aagcggcgca aagcatcgtc ttcggcgggg tgcgcgcgcg cagccaggac    1440
   ctcgacgcga tctggcgcgg tttctatatc gccggcgatc cggcgctggc ctacggctac    1500
   gcccaggacc aggaacccga cgcgcgcggc cggatccgca acggtgccct gctgcgggtc    1560
   tatgtgccgc gctcgagcct gccgggcttc taccgcaccg gcctgaccct ggccgcgccg    1620
   gaggcggcgg gcgaggtcga acggctgatc ggccatccgc tgccgctgcg cctggacgcc    1680
   atcaccggcc ccgaggagga aggcgggcgc gtgaccattc tcggctggcc gctggccgag    1740
   cgcaccgtgg tgattccctc ggcgatcccc accgacccgc gcaacgtcgg cggcgacctc    1800
   gacccgtcca gcatccccga caaggaacag gcgatcagcg ccctgccgga ctacgccagc    1860
   cagcccggca aaccgccgcg cgaggacctg aaggatcaga acgcgacctc agccgttacc    1920
   gagtattacc tgaatcacgg cgaatggccc ggcaacaaca cttctgccgg cgtggcaacc    1980
   tcctctgaaa tcaaaggagg tggacaccac caccaccacc actaa                    2025
<210> 4
   <211> 674
   <212> PRT
   <213> artificial
<220>
   <223>
<400> 4
   Met Lys Lys Ile Trp Leu Ala Leu Ala Gly Leu Val Leu Ala Phe Ser
   1               5                   10                  15
   Ala Ser Ala Ala Glu Glu Ala Phe Asp Leu Trp Asn Glu Cys Ala Lys
               20                  25                  30
   Ala Cys Val Leu Asp Leu Lys Asp Gly Val Arg Ser Ser Arg Met Ser
           35                  40                  45
   Val Asp Pro Ala Ile Ala Asp Thr Asn Gly Gln Gly Val Leu His Tyr
       50                  55                  60
   Ser Met Val Leu Glu Gly Gly Asn Asp Ala Leu Lys Leu Ala Ile Asp
   65                  70                  75                  80
   Asn Ala Leu Ser Ile Thr Ser Asp Gly Leu Thr Ile Arg Leu Glu Gly
                   85                  90                  95
   Gly Val Glu Pro Asn Lys Pro Val Arg Tyr Ser Tyr Thr Arg Gln Ala
               100                 105                 110
   Arg Gly Ser Trp Ser Leu Asn Trp Leu Val Pro Ile Gly His Glu Lys
           115                 120                 125
   Pro Ser Asn Ile Lys Val Phe Ile His Glu Leu Asn Ala Gly Asn Gln
       130                 135                 140
   Leu Ser His Met Ser Pro Ile Tyr Thr Ile Glu Met Gly Asp Glu Leu
   145                 150                 155                 160
   Leu Ala Lys Leu Ala Arg Asp Ala Thr Phe Phe Val Arg Ala His Glu
                   165                 170                 175
   Ser Asn Glu Met Gln Pro Thr Leu Ala Ile Ser His Ala Gly Val Ser
               180                 185                 190
   Val Val Met Ala Gln Ala Gln Pro Arg Arg Glu Lys Arg Trp Ser Glu
           195                 200                 205
   Trp Ala Ser Gly Lys Val Leu Cys Leu Leu Asp Pro Leu Asp Gly Val
       210                 215                 220
   Tyr Asn Tyr Leu Ala Gln Gln Arg Cys Asn Leu Asp Asp Thr Trp Glu
   225                 230                 235                 240
   Gly Lys Ile Tyr Arg Val Leu Ala Gly Asn Pro Ala Lys His Asp Leu
                   245                 250                 255
   Asp Ile Lys Pro Thr Val Ile Ser His Arg Leu His Phe Pro Glu Gly
               260                 265                 270
   Gly Ser Leu Ala Ala Leu Thr Ala His Gln Ala Cys His Leu Pro Leu
           275                 280                 285
   Glu Thr Phe Thr Arg His Arg Gln Pro Arg Gly Trp Glu Gln Leu Glu
       290                 295                 300
   Gln Cys Gly Tyr Pro Val Gln Arg Leu Val Ala Leu Tyr Leu Ala Ala
   305                 310                 315                 320
   Arg Leu Ser Trp Asn Gln Val Asp Gln Val Ile Gly Asn Ala Leu Ala
                   325                 330                 335
   Ser Pro Gly Ser Gly Gly Asp Leu Gly Glu Ala Ile Arg Glu Gln Pro
               340                 345                 350
   Glu Gln Ala Arg Leu Ala Leu Thr Leu Ala Ala Ala Glu Ser Glu Arg
           355                 360                 365
   Phe Val Arg Gln Gly Thr Gly Asn Asp Glu Ala Gly Ala Ala Ser Ala
       370                 375                 380
   Asp Val Val Ser Leu Thr Cys Pro Val Ala Ala Gly Glu Cys Ala Gly
   385                 390                 395                 400
   Pro Ala Asp Ser Gly Asp Ala Leu Leu Glu Arg Asn Tyr Pro Thr Gly
                   405                 410                 415
   Ala Glu Phe Leu Gly Asp Gly Gly Asp Ile Ser Phe Ser Thr Arg Gly
               420                 425                 430
   Thr Gln Asn Trp Thr Val Glu Arg Leu Leu Gln Ala His Arg Gln Leu
           435                 440                 445
   Glu Glu Arg Gly Tyr Val Phe Val Gly Tyr His Gly Thr Phe Leu Glu
       450                 455                 460
   Ala Ala Gln Ser Ile Val Phe Gly Gly Val Arg Ala Arg Ser Gln Asp
   465                 470                 475                 480
   Leu Asp Ala Ile Trp Arg Gly Phe Tyr Ile Ala Gly Asp Pro Ala Leu
                   485                 490                 495
   Ala Tyr Gly Tyr Ala Gln Asp Gln Glu Pro Asp Ala Arg Gly Arg Ile
               500                 505                 510
   Arg Asn Gly Ala Leu Leu Arg Val Tyr Val Pro Arg Ser Ser Leu Pro
           515                 520                 525
   Gly Phe Tyr Arg Thr Gly Leu Thr Leu Ala Ala Pro Glu Ala Ala Gly
       530                 535                 540
   Glu Val Glu Arg Leu Ile Gly His Pro Leu Pro Leu Arg Leu Asp Ala
   545                 550                 555                 560
   Ile Thr Gly Pro Glu Glu Glu Gly Gly Arg Val Thr Ile Leu Gly Trp
                   565                 570                 575
   Pro Leu Ala Glu Arg Thr Val Val Ile Pro Ser Ala Ile Pro Thr Asp
               580                 585                 590
   Pro Arg Asn Val Gly Gly Asp Leu Asp Pro Ser Ser Ile Pro Asp Lys
           595                 600                 605
   Glu Gln Ala Ile Ser Ala Leu Pro Asp Tyr Ala Ser Gln Pro Gly Lys
       610                 615                 620
   Pro Pro Arg Glu Asp Leu Lys Asp Gln Asn Ala Thr Ser Ala Val Thr
   625                 630                 635                 640
   Glu Tyr Tyr Leu Asn His Gly Glu Trp Pro Gly Asn Asn Thr Ser Ala
                   645                 650                 655
   Gly Val Ala Thr Ser Ser Glu Ile Lys Gly Gly Gly His His His His
               660                 665                 670
   His His
<210> 5
   <211> 501bp
   <212> DNA
   <213> artificial
<220>
   <223>
<400> 5
   atgaagaaaa tttggctggc cttagccggc ctggttctgg cattcagcgc cagcgcaacc      60
   ccgcagaaca tcaccgacct gtgcgccgag taccacaaca cccaaattta taccctgaac     120
   gacaaaattt ttagctacac cgagagcctg gcaggcaagc gcgagatggc catcatcacc     180
   ttcaagaacg gcgccatttt ccaggtggag gtgccgggca gccagcacat cgacagtcag     240
   aagaaggcca tcgagcgcat gaaggacacc ctgcgcatcg cctacctgac cgaggccaag     300
   gtggagaagc tgtgcgtgtg gaacaacaag accccgcacg ccatcgccgc aatcagcatg     360
   gccaacgacc agaacgccac cagcgccgtg accgagtact atctgaacca tggcgagtgg     420
   ccgggtaata acaccagcgc cggcgtggcc acaagcagtg agatcaaggg cggcggatcc     480
   caccatcacc accaccatta a                                                501
<210> 6
   <211> 166
   <212> PRT
   <213> artificial
<220>
   <223>
<400> 6
   Met Lys Lys Ile Trp Leu Ala Leu Ala Gly Leu Val Leu Ala Phe Ser
   1               5                   10                  15
   Ala Ser Ala Thr Pro Gln Asn Ile Thr Asp Leu Cys Ala Glu Tyr His
               20                  25                  30
   Asn Thr Gln Ile Tyr Thr Leu Asn Asp Lys Ile Phe Ser Tyr Thr Glu
           35                  40                  45
   Ser Leu Ala Gly Lys Arg Glu Met Ala Ile Ile Thr Phe Lys Asn Gly
       50                  55                  60
   Ala Ile Phe Gln Val Glu Val Pro Gly Ser Gln His Ile Asp Ser Gln
   65                  70                  75                  80
   Lys Lys Ala Ile Glu Arg Met Lys Asp Thr Leu Arg Ile Ala Tyr Leu
                   85                  90                  95
   Thr Glu Ala Lys Val Glu Lys Leu Cys Val Trp Asn Asn Lys Thr Pro
               100                 105                 110
   His Ala Ile Ala Ala Ile Ser Met Ala Asn Asp Gln Asn Ala Thr Ser
           115                 120                 125
   Ala Val Thr Glu Tyr Tyr Leu Asn His Gly Glu Trp Pro Gly Asn Asn
       130                 135                 140
   Thr Ser Ala Gly Val Ala Thr Ser Ser Glu Ile Lys Gly Gly Gly Ser
   145                 150                 155                 160
   His His His His His His
                   165
<210> 7
   <211> 1815bp
   <212> DNA
   <213> artificial
<220>
   <223>
<400> 7
   atgcccgctg aaacgaccgt atccggcgcg caccccgccg ccaaactgcc gatttacatc      60
   ctgccctgct tcctttggat aggcatcgtc ccctttacct tcgcgctcaa actgaaaccg     120
   tcgcccgact tttaccacga tgccgccgcc gcagccggcc tgattgtcct gttgttcctc     180
   acggcaggaa aaaaactgtt tgatgtcaaa atccccgcca tcagcttcct tctgtttgca     240
   atggcggcgt tttggtatct tcaggcacgc ctgatgaacc tgatttaccc cggtatgaac     300
   gacatcgtct cttggatttt catcttgctc gccgtcagcg cgtgggcctg ccggagcttg     360
   gtcgcacact tcggacaaga acgcatcgtg accctgtttg cctggtcgct gcttatcggc     420
   tccctgcttc aatcctgcat cgtcgtcatc cagtttgccg gctgggaaga cacccctctg     480
   tttcaaaaca tcatcgttta cagcgggcaa ggcgtaatcg gacacatcgg gcagcgcaac     540
   aacctcggac actacctcat gtggggcata ctcgccgccg cctacctcaa cggacaacga     600
   aaaatccccg ccgccctcgg cgtaatctgc ctgattatgc agaccgccgt tttaggtttg     660
   gtcaactcgc gcaccatctt gacctacata gccgccatcg ccctcatcct tcccttctgg     720
   tatttccgtt cggacaaatc caacaggcgg acgatgctcg gcatagccgc agccgtattc     780
   cttaccgcgc tgttccaatt ttccatgaac accattctgg aaacctttac tggcatccgc     840
   tacgaaactg ccgtcgaacg cgtcgccaac ggcggtttca cagacttgcc gcgccaaatc     900
   gaatggaata aagcccttgc cgccttccag tccgccccga tattcgggca cggctggaac     960
   agttttgccc aacaaacctt cctcatcaat gccgaacagc acaacatata cgacaacctc    1020
   ctcagcaact tgttcaccca ttcccacaac atcgtcctcc aactccttgc agagatggga    1080
   atcagcggca cgcttctggt tgccgcaacc ctgctgacgg gcattgccgg gctgcttaaa    1140
   cgccccctga cccccgcatc gcttttccta atctgcacgc ttgccgtcag tatgtgccac    1200
   agtatgctcg aatatccttt gtggtatgtc tatttcctca tccctttcgg actgatgctc    1260
   ttcctgtccc ccgcagaggc ttcagacggc atcgccttca aaaaagccgc caatctcggc    1320
   atactgaccg cctccgccgc catattcgca ggattgctgc acttggactg gacatacacc    1380
   cggctggtta acgccttttc ccccgccact gacgacagtg ccaaaaccct caaccggaaa    1440
   atcaacgagt tgcgctatat ttccgcaaac agtccgatgc tgtcctttta tgccgacttc    1500
   tccctcgtaa acttcgccct gccggaatac cccgaaaccc agacttgggc ggaagaagca    1560
   accctcaaat cactaaaata ccgcccccac tccgccacct accgcatcgc cctctacctg    1620
   atgcggcaag gcaaagttgc agaagcaaaa caatggatgc gggcgacaca gtcctattac    1680
   ccctacctga tgccccgata cgccgacgaa atccgcaaac tgcccgtatg ggcgccgctg    1740
   ctacccgaac tgctcaaaga ctgcaaagcc ttcgccgccg cgcccggtca tccggaagca    1800
   aaaccctgca aatga                                                     1815
<210> 8
   <211> 604
   <212> PRT
   <213> artificial
<220>
   <223>
<400> 8
   Met Pro Ala Glu Thr Thr Val Ser Gly Ala His Pro Ala Ala Lys Leu
   1               5                   10                  15
   Pro Ile Tyr Ile Leu Pro Cys Phe Leu Trp Ile Gly Ile Val Pro Phe
               20                  25                  30
   Thr Phe Ala Leu Lys Leu Lys Pro Ser Pro Asp Phe Tyr His Asp Ala
           35                  40                  45
   Ala Ala Ala Ala Gly Leu Ile Val Leu Leu Phe Leu Thr Ala Gly Lys
       50                  55                  60
   Lys Leu Phe Asp Val Lys Ile Pro Ala Ile Ser Phe Leu Leu Phe Ala
   65                  70                  75                  80
   Met Ala Ala Phe Trp Tyr Leu Gln Ala Arg Leu Met Asn Leu Ile Tyr
                   85                  90                  95
   Pro Gly Met Asn Asp Ile Val Ser Trp Ile Phe Ile Leu Leu Ala Val
               100                 105                 110
   Ser Ala Trp Ala Cys Arg Ser Leu Val Ala His Phe Gly Gln Glu Arg
           115                 120                 125
   Ile Val Thr Leu Phe Ala Trp Ser Leu Leu Ile Gly Ser Leu Leu Gln
       130                 135                 140
   Ser Cys Ile Val Val Ile Gln Phe Ala Gly Trp Glu Asp Thr Pro Leu
   145                 150                 155                 160
   Phe Gln Asn Ile Ile Val Tyr Ser Gly Gln Gly Val Ile Gly His Ile
                   165                 170                 175
   Gly Gln Arg Asn Asn Leu Gly His Tyr Leu Met Trp Gly Ile Leu Ala
               180                 185                 190
   Ala Ala Tyr Leu Asn Gly Gln Arg Lys Ile Pro Ala Ala Leu Gly Val
           195                 200                 205
   Ile Cys Leu Ile Met Gln Thr Ala Val Leu Gly Leu Val Asn Ser Arg
       210                 215                 220
   Thr Ile Leu Thr Tyr Ile Ala Ala Ile Ala Leu Ile Leu Pro Phe Trp
   225                 230                 235                 240
   Tyr Phe Arg Ser Asp Lys Ser Asn Arg Arg Thr Met Leu Gly Ile Ala
                   245                 250                 255
   Ala Ala Val Phe Leu Thr Ala Leu Phe Gln Phe Ser Met Asn Thr Ile
               260                 265                 270
   Leu Glu Thr Phe Thr Gly Ile Arg Tyr Glu Thr Ala Val Glu Arg Val
           275                 280                 285
   Ala Asn Gly Gly Phe Thr Asp Leu Pro Arg Gln Ile Glu Trp Asn Lys
       290                 295                 300
   Ala Leu Ala Ala Phe Gln Ser Ala Pro Ile Phe Gly His Gly Trp Asn
   305                 310                 315                 320
   Ser Phe Ala Gln Gln Thr Phe Leu Ile Asn Ala Glu Gln His Asn Ile
                   325                 330                 335
   Tyr Asp Asn Leu Leu Ser Asn Leu Phe Thr His Ser His Asn Ile Val
               340                 345                 350
   Leu Gln Leu Leu Ala Glu Met Gly Ile Ser Gly Thr Leu Leu Val Ala
           355                 360                 365
   Ala Thr Leu Leu Thr Gly Ile Ala Gly Leu Leu Lys Arg Pro Leu Thr
       370                 375                 380
   Pro Ala Ser Leu Phe Leu Ile Cys Thr Leu Ala Val Ser Met Cys His
   385                 390                 395                 400
   Ser Met Leu Glu Tyr Pro Leu Trp Tyr Val Tyr Phe Leu Ile Pro Phe
                   405                 410                 415
   Gly Leu Met Leu Phe Leu Ser Pro Ala Glu Ala Ser Asp Gly Ile Ala
               420                 425                 430
   Phe Lys Lys Ala Ala Asn Leu Gly Ile Leu Thr Ala Ser Ala Ala Ile
           435                 440                 445
   Phe Ala Gly Leu Leu His Leu Asp Trp Thr Tyr Thr Arg Leu Val Asn
       450                 455                 460
   Ala Phe Ser Pro Ala Thr Asp Asp Ser Ala Lys Thr Leu Asn Arg Lys
   465                 470                 475                 480
   Ile Asn Glu Leu Arg Tyr Ile Ser Ala Asn Ser Pro Met Leu Ser Phe
                   485                 490                 495
   Tyr Ala Asp Phe Ser Leu Val Asn Phe Ala Leu Pro Glu Tyr Pro Glu
               500                 505                 510
   Thr Gln Thr Trp Ala Glu Glu Ala Thr Leu Lys Ser Leu Lys Tyr Arg
           515                 520                 525
   Pro His Ser Ala Thr Tyr Arg Ile Ala Leu Tyr Leu Met Arg Gln Gly
       530                 535                 540
   Lys Val Ala Glu Ala Lys Gln Trp Met Arg Ala Thr Gln Ser Tyr Tyr
   545                 550                 555                 560
   Pro Tyr Leu Met Pro Arg Tyr Ala Asp Glu Ile Arg Lys Leu Pro Val
                   565                 570                 575
   Trp Ala Pro Leu Leu Pro Glu Leu Leu Lys Asp Cys Lys Ala Phe Ala
               580                 585                 590
   Ala Ala Pro Gly His Pro Glu Ala Lys Pro Cys Lys
           595                 600
<210> 9
   <211> 699bp
   <212> DNA
   <213> artificial
<220>
   <223>
<400> 9
   atgaagaaaa tttggctggc cttagccggc ctggttctgg cattcagcgc cagcgcaacc      60
   ccgcagaaca tcaccgacct gtgcgccgag taccacaaca cccaaattta taccctgaac     120
   gacaaaattt ttagctacac cgagagcctg gcaggcaagc gcgagatggc catcatcacc     180
   ttcaagaacg gcgccatttt ccaggtggag gtgccgggca gccagcacat cgacagtcag     240
   aagaaggcca tcgagcgcat gaaggacacc ctgcgcatcg cctacctgac cgaggccaag     300
   gtggagaagc tgtgcgtgtg gaacaacaag accccgcacg ccatcgccgc aatcagcatg     360
   gccaacgacc agaacgccac cagcgccgtg accgagtact atctgaacca tggcgagtgg     420
   ccgggtaata acaccagcgc cggcgtggcc acaagcagtg agatcaaggg cggcggatct     480
   agcgccgtga ccgagtacta tctgaaccat ggcgagtggc cgggtaataa caccagcgcc     540
   ggcgtggcca caagcagtga gatcaagggc ggcggatcta gcgccgtgac cgagtactat     600
   ctgaaccatg gcgagtggcc gggtaataac accagcgccg gcgtggccac aagcagtgag     660
   atcaagggcg gcggatccca ccatcaccac caccattaa                            699
<210> 10
   <211> 232
   <212> PRT
   <213> artificial
<220>
   <223>
<400> 10
   Met Lys Lys Ile Trp Leu Ala Leu Ala Gly Leu Val Leu Ala Phe Ser
   1               5                   10                  15
   Ala Ser Ala Thr Pro Gln Asn Ile Thr Asp Leu Cys Ala Glu Tyr His
               20                  25                  30
   Asn Thr Gln Ile Tyr Thr Leu Asn Asp Lys Ile Phe Ser Tyr Thr Glu
           35                  40                  45
   Ser Leu Ala Gly Lys Arg Glu Met Ala Ile Ile Thr Phe Lys Asn Gly
       50                  55                  60
   Ala Ile Phe Gln Val Glu Val Pro Gly Ser Gln His Ile Asp Ser Gln
   65                  70                  75                  80
   Lys Lys Ala Ile Glu Arg Met Lys Asp Thr Leu Arg Ile Ala Tyr Leu
                   85                  90                  95
   Thr Glu Ala Lys Val Glu Lys Leu Cys Val Trp Asn Asn Lys Thr Pro
               100                 105                 110
   His Ala Ile Ala Ala Ile Ser Met Ala Asn Asp Gln Asn Ala Thr Ser
           115                 120                 125
   Ala Val Thr Glu Tyr Tyr Leu Asn His Gly Glu Trp Pro Gly Asn Asn
       130                 135                 140
   Thr Ser Ala Gly Val Ala Thr Ser Ser Glu Ile Lys Gly Gly Gly Ser
   145                 150                 155                 160
   Ser Ala Val Thr Glu Tyr Tyr Leu Asn His Gly Glu Trp Pro Gly Asn
                   165                 170                 175
   Asn Thr Ser Ala Gly Val Ala Thr Ser Ser Glu Ile Lys Gly Gly Gly
               180                 185                 190
   Ser Ser Ala Val Thr Glu Tyr Tyr Leu Asn His Gly Glu Trp Pro Gly
           195                 200                 205
   Asn Asn Thr Ser Ala Gly Val Ala Thr Ser Ser Glu Ile Lys Gly Gly
               210                 215                 220
   Gly Ser His His His His His His
   225     230
<210> 11
   <211> 2102bp
   <212> DNA
   <213> artificial
<220>
   <223>
<400> 11
   cagttgctgg ctaattatca gtcagtgcct aataatatta tctccgctat cgttgaagca      60
   aaccgtacgc gtaaagattt tattgccgat tctatccttg ccagaaagcc taaagtggtg     120
   ggcatttatc gcctgattat gaagagtggc tccgataact tccgcgcctc atcgattcag     180
   gggattatga aacgtattaa agcgaaaggc gtagaggtca tcatctatga gccggtaatg      240
   caagaagaaa ctttctttaa ctcacgtctt gaacgtgatt tgcactgctt taaacagcag     300
   gcggatgtca ttatttccaa tcggatggcg gcagagcttt tggatgtcgc tgaaaaagtg     360
   tatacccgcg atcttttcgg tagtgattaa taaggcaatc ggggctgatg agtcccaatg     420
   atttattgac caaatggaaa taatgtctga tttttatcat taatcctatg gcatatattt     480
   tctttatgac tacactgtct ccagcttcat ccttttttta gttagggtat ctatgacaag      540
   cgattgtgta ggctggagct gcttcgaagt tcctatactt tctagagaat aggaacttcg     600
   gaataggaac ttcatttaaa tggcgcgcct tacgccccgc cctgccactc atcgcagtac     660
   tgttgtattc attaagcatc tgccgacatg gaagccatca caaacggcat gatgaacctg     720
   aatcgccagc ggcatcagca ccttgtcgcc ttgcgtataa tatttgccca tggtgaaaac     780
   gggggcgaag aagttgtcca tattggccac gtttaaatca aaactggtga aactcaccca     840
   gggattggct gagacgaaaa acatattctc aataaaccct ttagggaaat aggccaggtt     900
   ttcaccgtaa cacgccacat cttgcgaata tatgtgtaga aactgccgga aatcgtcgtg     960
   gtattcactc cagagcgatg aaaacgtttc agtttgctca tggaaaacgg tgtaacaagg    1020
   gtgaacacta tcccatatca ccagctcacc gtctttcatt gccatacgta attccggatg    1080
   agcattcatc aggcgggcaa gaatgtgaat aaaggccgga taaaacttgt gcttattttt    1140
   ctttacggtc tttaaaaagg ccgtaatatc cagctgaacg gtctggttat aggtacattg    1200
   agcaactgac tgaaatgcct caaaatgttc tttacgatgc cattgggata tatcaacggt    1260
   ggtatatcca gtgatttttt tctccatttt agcttcctta gctcctgaaa atctcgacaa    1320
   ctcaaaaaat acgcccggta gtgatcttat ttcattatgg tgaaagttgg aacctcttac    1380
   gtgccgatca acgtctcatt ttcgccaaaa gttggcccag ggcttcccgg tatcaacagg    1440
   gacaccagga tttatttatt ctgcgaagtg atcttccgtc acaggtaggc gcgccgaagt    1500
   tcctatactt tctagagaat aggaacttcg gaataggaac taaggaggat attcatatgg    1560
   accatggcta attcccatgt cagccgttaa gcttctttgc cggatggtgg tcggcttcga    1620
   aaggtaaaat ttctcattaa aaaaccgggc attgcccggt ttttttacgc ttattgatga    1680
   cgcttacgaa gattgtcgat cacggtggtg aggttgagat cctgatcctg caacagcacc    1740
   agcaggtggt acatcaaatc agacgcttca ttggttaatt caaaacgatc attgacggtg    1800
   gccgccagcg cggtttctac accttcttcg ccaacttttt gcgcaatacg tttggtaccg    1860
   ctggcgtaca gtttcgccgt gtaagagctg gccggatcgg cggttttgcg ctctgccagt    1920
   agctgctcca gttgatacag gaataaccac tgatggctgg cgtcgccaaa gcagctgctg    1980
   gtacctttgt ggcaggtcgg cccgacaggg tttgccagca ccagcagtgt atcgttgtcg    2040
   caatctggcg caatgctgac gacattcagc acatggcctg aggtttcgcc tttggtccat    2100
   aa                                                                   2102
<210> 12
   <211> 2549bp
   <212> DNA
   <213> artificial
<220>
   <223>
<400> 12
   ggatccaggc tttgactatg tggatgtgtt accgcgcatg agcctggagg aggtcgccag      60
   agtgctggct ggcgcaaaat ttgtcgtatc ggttgatacc ggcctgagcc atctttccgc     120
   ggcgctcgac agaccgaata ttacgctata tggcccaacg gaccctgggt taattggagg     180
   ttatgggaag aaccaaatgg catgctgctc accagaacaa aacctggcga atttagatgc     240
   cacaagcgta tttggaaaga ttcattaaag agactctgtc tcatcccaaa cctattgtgg     300
   agaaaagatg ctaaccacat cattaacgtt aaataaagag aaatggaagc cgatctggaa     360
   taaagcgctg gtttttcttt ttgttgccac gtattttctg gatggtatta cgcgttataa     420
   acatttgata atcatactta tggttatcac cgcgatttat caggtctcac gctcaccgaa     480
   aagtttcccc cctcttttca aaaatagcgt attttatagc gtagcagtat tatcattaat      540
   ccttgtttat tccatactca tatcgccaga tgtcgacgtg taggctggag ctgcttcgaa      600
   gttcctatac tttctagaga ataggaactt cggaatagga acttcaagat cccctcacgc      660
   tgccgcaagc actcagggcg caagggctgc taaaggaagc ggaacacgta gaaagccagt      720
   ccgcagaaac ggtgctgacc ccggatgaat gtcagctact gggctatctg gacaagggaa      780
   aacgcaagcg caaagagaaa gcaggtagct tgcagtgggc ttacatggcg atagctagac      840
   tgggcggttt tatggacagc aagcgaaccg gaattgccag ctggggcgcc ctctggtaag      900
   gttgggaagc cctgcaaagt aaactggatg gctttcttgc cgccaaggat ctgatggcgc      960
   aggggatcaa gatctgatca agagacagga tgaggatcgt ttcgcatgat tgaacaagat     1020
   ggattgcacg caggttctcc ggccgcttgg gtggagaggc tattcggcta tgactgggca     1080
   caacagacaa tcggctgctc tgatgccgcc gtgttccggc tgtcagcgca ggggcgcccg     1140
   gttctttttg tcaagaccga cctgtccggt gccctgaatg aactgcagga cgaggcagcg     1200
   cggctatcgt ggctggccac gacgggcgtt ccttgcgcag ctgtgctcga cgttgtcact     1260
   gaagcgggaa gggactggct gctattgggc gaagtgccgg ggcaggatct cctgtcatct     1320
   caccttgctc ctgccgagaa agtatccatc atggctgatg caatgcggcg gctgcatacg     1380
   cttgatccgg ctacctgccc attcgaccac caagcgaaac atcgcatcga gcgagcacgt     1440
   actcggatgg aagccggtct tgtcgatcag gatgatctgg acgaagagca tcaggggctc     1500
   gcgccagccg aactgttcgc caggctcaag gcgcgcatgc ccgacggcga ggatctcgtc     1560
   gtgacccatg gcgatgcctg cttgccgaat atcatggtgg aaaatggccg cttttctgga     1620
   ttcatcgact gtggccggct gggtgtggcg gaccgctatc aggacatagc gttggctacc     1680
   cgtgatattg ctgaagagct tggcggcgaa tgggctgacc gcttcctcgt gctttacggt     1740
   atcgccgctc ccgattcgca gcgcatcgcc ttctatcgcc ttcttgacga gttcttctga     1800
   gcgggactct ggggttcgaa atgaccgacc aagcgacgcc caacctgcca tcacgagatt     1860
   tcgattccac cgccgccttc tatgaaaggt tgggcttcgg aatcgttttc cgggacgccg     1920
   gctggatgat cctccagcgc ggggatctca tgctggagtt cttcgcccac cccagcttca     1980
   aaagcgctct gaagttccta tactttctag agaataggaa cttcggaata ggaactaagg     2040
   aggatattca tatggaccat ggctaattcc cataagcttt agtgcaggca tattgggtct     2100
   ggcgagcctg gcctatttat atggtgctat catcagggaa acagccagct ctaccttcag     2160
   gaaagtagag ataagcccct acaatgctca tctcttgcta tttttatctt tcgtcggttt     2220
   ttatatcgtt cgtggcaatt ttgaacaggt cgatattgct caaattggta tcattaccgg     2280
   ttttctgctg gcgctaagaa atagataaaa aacgcgctga tacttattac ggtatcagcg     2340
   cgttttccat catcaggact caatcactta tcaaaccagt ttttcatttg ttcctcgaaa      2400
   cgctgcgcta cattttccca actgtatttt gaaaacacca gggattttgc tttttcggca      2460
   atctggtggc gttccttatc agcaagcgca cggttaatat cattaattat actgtcgctc     2520
   gacataggtt ctgcgaggtg atactcgag                                       2549
<210> 13
   <211> 87bp
   <212> DNA
   <213> artificial
<220>
   <223>
<400> 13
   agcgccgtga ccgagtacta tctgaaccat ggcgagtggc cgggtaataa caccagcgcc       60
   ggcgtggcca caagcagtga gatcaag                                           87
<210> 14
   <211> 29
   <212> PRT
   <213> artificial
<220>
   <223>
<400> 14
   Ser Ala Val Thr Glu Tyr Tyr Leu Asn His Gly Glu Trp Pro Gly Asn
   1               5                   10                  15
   Asn Thr Ser Ala Gly Val Ala Thr Ser Ser Glu Ile Lys
               20                  25